(19) Europäisches Patentamt
European Patent Office
Office européen des brevets

(11) EP 4 143 545 B1

(12) **FASCICULE DE BREVET EUROPEEN**

(45) Date de publication et mention de la délivrance du brevet:
**26.08.2026 Bulletin 2026/35**

(21) Numéro de dépôt: **21732411.0**

(22) Date de dépôt: **30.04.2021**

(51) Classification Internationale des Brevets (IPC):
***G01N 21/65*** *(2006.01)* ***G01N 33/58*** *(2006.01)*
***C12Q 1/04*** *(2006.01)*

(52) Classification Coopérative des Brevets (CPC):
**G01N 33/54346; G01N 21/658; G01N 33/587;**
C12Q 1/04; Y02A 50/30

(86) Numéro de dépôt international:
**PCT/FR2021/050751**

(87) Numéro de publication internationale:
**WO 2021/219969 (04.11.2021 Gazette 2021/44)**

(54) **MÉTHODE DE DÉTECTION DE LA PRÉSENCE D'UN AGENT PATHOGÈNE DANS UN LIQUIDE BIOLOGIQUE PAR SPECTROSCOPIE RAMAN EXALTÉE DE SURFACE**

VERFAHREN ZUM NACHWEIS EINES PATHOGENEN WIRKSTOFFS IN EINER BIOLOGISCHEN FLÜSSIGKEIT MITTELS OBERFLÄCHENVERSTÄRKTER RAMAN-SPEKTROSKOPIE

METHOD FOR DETECTING THE PRESENCE OF A PATHOGENIC AGENT IN A BIOLOGICAL FLUID BY SURFACE ENHANCED RAMAN SPECTROSCOPY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.04.2020 FR 2004334**
**28.10.2020 FR 2011030**

(43) Date de publication de la demande:
**08.03.2023 Bulletin 2023/10**

(73) Titulaire: **SPORE BIOTECHNOLOGIES**
**92100 Boulogne-Billancourt (FR)**

(72) Inventeurs:
- **BOULANGER, Anthony François Michel Claude Dieudonné**
  **75116 Paris (FR)**
- **CASTELAIN, Sandrine**
  **80000 Amiens (FR)**
- **LEFRANC, Sandrine Godeliève Agnès**
  **78180 Montigny-le-Bretonneux (FR)**
- **GAUVAIN, Pierre Michel René**
  **28130 Saint-Piat (FR)**
- **PEREZ, Florent Jules Adrien**
  **75016 Paris (FR)**
- **EL MESSAOUDI, Sanaa**
  **78500 Sartrouville (FR)**
- **BANON, Alexandre Antoine Benjamin Marie**
  **75013 Paris (FR)**
- **GARSUAULT, Delphine Stéphanie Jeannine Marie-Thérèse**
  **75014 Paris (FR)**
- **GUEDET, Tiffany**
  **92000 Nanterre (FR)**
- **SCHMITT-BOULANGER, Marion**
  **75116 Paris (FR)**

(74) Mandataire: **Plasseraud IP**
**104 Rue de Richelieu**
**CS92104**
**75080 Paris Cedex 02 (FR)**

(56) Documents cités:
**EP-A1- 3 901 617 WO-A1-2021/203041**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **HUNTER ROBERT ET AL: "Optofluidic label-free SERS platform for rapid bacteria detection in serum", SENSORS AND ACTUATORS B: CHEMICAL, ELSEVIER BV, NL, vol. 300, 31 July 2019 (2019-07-31), XP085836144, ISSN: 0925-4005, [retrieved on 20190731], DOI: 10.1016/J.SNB.2019.126907**
- **MUNGROO NAWFAL ADAM ET AL: "SERS based point-of-care detection of food-borne pathogens", MIKROCHIMICA ACTA, SPRINGER VERLAG, VIENNA, AT, vol. 183, no. 2, 15 December 2015 (2015-12-15), pages 697 - 707, XP035887483, ISSN: 0026-3672, [retrieved on 20151215], DOI: 10.1007/S00604-015-1698-Y**
- **AKANNY ELIE ET AL: "Development of uncoated near-spherical gold nanoparticles for the label-free quantification ofLactobacillus rhamnosus GGby surface-enhanced Raman spectroscopy", ANALYTICAL AND BIOANALYTICAL CHEMISTRY, SPRINGER BERLIN HEIDELBERG, DE, vol. 411, no. 21, 17 June 2019 (2019-06-17), pages 5563 - 5576, XP036853507, ISSN: 1618-2642, [retrieved on 20190617], DOI: 10.1007/S00216-019-01938-4**

## Description

### Domaine technique

La présente invention concerne un kit pour la détection de la présence d'un agent pathogène par spectroscopie Raman exaltée de surface (en anglais Surface Enhanced Raman Spectroscopy ou SERS), l'utilisation dudit kit et une méthode de détection de la présence d'un agent pathogène par SERS. Cette méthode rapide et reproductible présente une haute sensibilité et spécificité. Le domaine de l'invention est plus particulièrement celui de la détection de la présence d'un agent pathogène dans un échantillon biologique.

### Etat de la technique antérieure

Divers agents pathogènes, dont les virus, sont responsables chaque année de nombreuses maladies humaines courantes, telles que les infections respiratoires hautes (rhinites, pharyngites), la grippe, les infections gastro-intestinales, ou encore les infections virales de la petite enfance telles que la varicelle, la rougeole et les oreillons. Certaines maladies virales ont un pouvoir de morbidité ou mortalité important telles que les fièvres hémorragiques (virus EBOLA, virus amaril), les encéphalites virales (virus de la rage, virus de la dengue, Herpes simplex virus, le poliovirus), et le syndrome d'immunodéficience acquise (SIDA). La rougeole et le cytomégalovirus peuvent provoquer de graves anomalies ou entrainer le décès chez les enfants à naître. Sur les 1 000 à 1 500 types de virus estimés, environ 250 provoquent des maladies chez l'homme. Plusieurs virus humains tels que le virus Epstein Barr, les papillomavirus et les virus des hépatites B et C ont également été associés au développement de cancers.

Apparue en Chine fin 2019, la maladie Covid-19 est un syndrome respiratoire aigu sévère causé par le SARS-CoV-2, un virus appartenant à la famille des coronavirus. Ces derniers, très fréquents, peuvent aussi bien provoquer un simple rhume comme une infection respiratoire des voies basses de type pneumonie, à l'origine d'épidémies mortelles comme celles du syndrome respiratoire aigu sévère (SARS-CoV en 2003), du Syndrome Respiratoire du Moyen-Orient (MERS-CoV en 2012), et maintenant du Covid-19 (SARS-CoV-2).

Les méthodes de diagnostic viral actuelles impliquent :

(i) soit la recherche de la particule virale ou de l'un de ses composants (antigènes viraux, génome viral ou bien une propriété enzymatique d'une protéine virale) et correspond au diagnostic direct ; cette recherche utilise des tests de détection rapides pour les antigènes ou des techniques de biologie moléculaire pour le génome (PCR ou RT-PCR selon la nature du génome ADN ou ARN)

(ii) soit la recherche de la réponse de l'hôte vis-à-vis du virus, correspondant à la détection d'anticorps spécifiques du virus recherché. Cette recherche se fait principalement par des techniques d'immunochimie le plus souvent automatisées tels que l'ELISA (« *enzyme-linked-immuno-sorbent-assay* »).

La recherche du génome viral, qui est une technique sensible et spécifique largement déployée, nécessite cependant un équipement lourd et coûteux et est souvent chronophage.

Actuellement, pour le SARS-CoV-2, seul un test de biologie moléculaire par RT-PCR sur un prélèvement naso-pharyngé permet de confirmer une infection au SARS-CoV-2.

De nombreux tests antigéniques ou sérologiques sont en cours de déploiement et de validation. Ces tests ciblent les protéines de surface Spike du virus ou permettent de mettre en évidence la présence ou non d'immunoglobulines IgG et/ou IgM selon les kits.

La spectroscopie Raman est une méthode d'analyse chimique non invasive. C'est une spectroscopie vibrationnelle à l'instar de la spectroscopie infrarouge (IR) qui fournit une caractérisation simultanée de la composition chimique d'un matériau, de son environnement ou encore de son degré d'oxydation. C'est une technique initialement peu sensible mais sa faible sensibilité a été compensée par l'introduction de la spectroscopie Raman exaltée de surface (en anglais *« Surface Enhanced Raman Spectroscopy »,* ou SERS). Cette méthodologie repose sur l'utilisation de substrats nanostructurés ou sous forme de nanoparticules métalliques. Les électrons libres du métal oscillent dans ces nanostructures à la résonance des plasmons de surface *(« Localized Surface Plasmon Resonance »,* ou LSPR), donnant lieu à une forte amplification localisée du signal Raman de composés à proximité, jusqu'à $10^{12}$, permettant d'aller jusqu'à la détection de molécules uniques.

Pour augmenter la spécificité de la technique, il est possible de fonctionnaliser le substrat utilisé. Ces nanoparticules fonctionnalisées sont destinées à se lier spécifiquement aux molécules biologiques (telles que les protéines et les acides nucléiques) provenant d'un agent pathogène dont on cherche à déterminer s'il est présent dans l'échantillon. Dans ce cas, la présence de pics dans le spectre SERS obtenu à partir de l'échantillon traduit la présence de l'agent pathogène recherché. À l'inverse, les molécules qui ne proviennent pas de l'agent pathogène recherché ne voient pas leur signal Raman amplifié, et ne contribuent que de façon marginale au signal SERS. En d'autres termes, en cas de présence

de l'agent pathogène dans l'échantillon, le signal de spectrométrie SERS obtenu sur la base dudit échantillon ne comprend quasiment que les contributions des molécules provenant dudit agent pathogène.

De cette façon, une complexité excessive du spectre SERS, qui rendrait très difficile son interprétation, est évitée. En effet, sans la fonctionnalisation des nanoparticules métalliques, tous les composés présents dans l'échantillon sont susceptibles de contribuer au spectre Raman dans des proportions comparables : il en résulterait un spectre Raman dont la complexité rendrait très difficile son interprétation.

Toutefois, un tel procédé de détection ne donne pas entière satisfaction.

En effet, du fait de la fonctionnalisation décrite ci-dessus, le procédé de détection de l'état de la technique n'est susceptible de détecter qu'un unique agent pathogène auquel les nanoparticules métalliques sont spécifiques, du fait de leur fonctionnalisation. La détection d'un nouvel agent pathogène requiert donc l'utilisation de nanoparticules métalliques différentes, spécifiques à ce nouvel agent pathogène. Il en résulte un procédé de détection peu versatile, fastidieux à mettre en œuvre.

En outre, le procédé de détection de l'état de la technique est sensible aux variants et aux mutations : en d'autres termes, il est susceptible de ne pas détecter un variant ou un mutant d'un agent pathogène donné, si la mutation impacte un site sur lequel il est prévu que les nanoparticules métalliques fonctionnalisées se fixent.

Le document Hunter Robert et al. : « optofluidic label-free SERS platform for rapid bacteria detection in serum", sensors and actuators b: chemical, Elsevier bv, 31 juillet 2019 décrit une plateforme de détection Raman qui effectue un comptage automatique par un algorithme d'apprentissage de la machine à vecteur de support ; le document Mungroo Nawfal Adam et al. : « SERS based point of care detection of food-borne pathogens », mikrochimica acta springer verlag, Vienna, at, 15 décembre 2015 décrit une plateforme de détection des bactéries en utilisant des nanoparticules d'argent; WO2021/20304 1A1 porte sur des méthodes de détection des pathogènes basés sur la spectroscopie Raman ; et EP3901617A1 concerne la détection des agents tels que des bactéries ou des virus en utilisant spectroscopie Raman.

Par conséquent, il existe un besoin de mettre à disposition une méthode de détection fiable et rapide pour détecter des agents pathogènes, par exemple des virus, notamment le SARS-Cov-2.

Un but de l'invention est donc de proposer une méthode de détection qui soit plus rapide, plus reproductible, plus sensible et/ou plus spécifique que le procédé de l'état de la technique, tout en étant plus versatile (c'est-à-dire universelle), plus facile à mettre en œuvre et moins sensible aux variants et aux mutations des agents pathogènes.

Le but de la présente invention est également de fournir :

- un produit programme d'ordinateur ;
- un kit pour la détection d'un agent pathogène par spectroscopie Raman exaltée de surface (SERS) ; et/ou
- son utilisation pour la détection d'un agent pathogène par spectroscopie Raman exaltée de surface (SERS)

qui soi(en)t susceptible(s) de résoudre les problèmes décrits ci-dessus.

## Exposé de l'invention

La présente invention a donc pour objet une méthode de détection du SARS-Cov-2 comme définie dans la revendication 1.

Le recours à des nanoparticules métalliques natives résulte en un signal SERS qui est représentatif de la présence ou non de tout agent pathogène, sans nécessité de recourir à un lot de nanoparticules métalliques fonctionnalisées spécifiquement pour un agent pathogène donné.

Bien que le signal de spectroscopie SERS obtenu grâce à la méthode selon l'invention soit très complexe (empêchant ainsi son analyse par un opérateur), le recours à un modèle de classification permet, de façon fiable, d'extraire du signal SERS acquis les caractéristiques permettant de conclure à la présence ou non d'un agent pathogène donné dans l'échantillon.

Par « nanoparticule métallique native », il est entendu, au sens de la présente invention, une nanoparticule métallique non fonctionnalisée, c'est-à-dire une nanoparticule métallique nue.

La présente invention a également pour objet un kit pour la détection de la présence du SARS-Cov-2 comme défini dans la revendication 9.

Les inventeurs ont montré que des nanoparticules métalliques non magnétiques en suspension permettaient d'obtenir facilement et rapidement des données SERS faciles à interpréter et reproductibles.

De façon très avantageuse, dans tous les exemples décrits, et dans toutes les variantes envisagées, les nanoparticules métalliques non magnétiques sont des nanoparticules métalliques natives non magnétiques.

Les nanoparticules métalliques non magnétiques comprennent, de préférence, un mélange de nanoparticules non magnétiques métalliques d'un premier métal et de nanoparticules non magnétiques d'un second métal, les nanoparticules du premier métal étant différentes des nanoparticules du second métal. Ceci est avantageux, dans la mesure où des nanoparticules réalisées dans des métaux différents sont susceptibles d'amplifier les photons de diffusion

Raman dans des plages spectrales différentes. Il en résulte un spectre SERS plus riche, ce qui est susceptible de faciliter la détection d'un agent pathogène recherché.

Conformément à l'invention, le tampon de lyse, qui est optionnel, peut être tout tampon connu de l'homme du métier apte à solubiliser les protéines des agents pathogènes telles que la capside pour les virus, les protéines membranaires ou d'enveloppe pour les champignons, les levures, les bactéries ou les virus et à libérer l'ARN ou l'ADN de l'échantillon. Ce tampon doit être compatible avec la technique SERS. On peut citer à titre d'exemple des tampons de lyse à base de Tris (également appelé tris(hydroxyméthyl)aminométhane), EDTA (également appelé acide éthylène-diaminetétraacétique), d'HEPES (également appelé acide 4-(2-hydroxyéthyl)-1-pipérazine éthane sulfonique) ou de SDS (également appelé dodécylsulfate de sodium). Conformément à l'invention, l'échantillon peut être choisi parmi le sang, le plasma, la salive, les larmes, le liquide naso-pharyngé, la sueur, les urines, la lymphe, le liquide céphalorachidien, du tissu humain ou animal ou des cellules humaines ou animales. L'échantillon peut être également tout liquide comme de l'eau du robinet ou de rivière ou ayant servi à rincer une surface susceptible d'être contaminée.

Dans un mode de réalisation avantageux de l'invention, les nanoparticules métalliques non magnétiques présentent un diamètre moyen compris entre 50 et 200 nm (en particulier 50, 60, 70, 80, 90, 100 et/ou 200 nm), avantageusement compris entre 100 et 200 nm (en particulier 100, 150 et/ou 200 nm), encore plus avantageusement compris entre 100 et 150 nm (en particulier 110, 115, 120, 125, 130, 135, 140, 145 et/ou 150 nm). Un tel diamètre moyen est, par exemple, mesuré par résonance des plasmons de surface (ou SPR, de l'anglais *« Surface Plasmon Resonance »),* par microscope électronique à transmission (ou TEM, de l'anglais *« Transmission Electron Microscope »),* par diffusion dynamique de la lumière (ou DLS, de l'anglais *« Dynamic Light Scattering »)* ou encore par électrophorèse capillaire de zone (ou CZE, de l'anglais *« Capillary Zone Electrophoresis »).*

Dans un autre mode de réalisation avantageux de l'invention, les nanoparticules métalliques non magnétiques sont des nanoparticules d'or, d'argent, de cuivre, de platine ou d'un alliage à base d'un de ces métaux. Dans le cas des mélanges de nanoparticules, les nanoparticules métalliques non magnétiques du premier métal sont de préférence des nanoparticules d'or et les nanoparticules métalliques non magnétiques du second métal sont de préférence des nanoparticules d'argent.

Conformément à l'invention les nanoparticules comprennent de préférence substantiellement un métal unique mais peuvent aussi comprendre des alliages de métaux, par exemple des alliages binaires.

Ces nanoparticules sont disponibles dans le commerce sous forme de solutions ou de suspension colloïdale.

Conformément à l'invention dans le cas des mélanges, le rapport entre les deux types de nanoparticules est de préférence compris entre 5/95 et 95/5 et avantageusement compris entre 40/60 et 60/40, en particulier 50/50. Ainsi il peut y avoir dans le mélange de 5% de particules d'or et 95% de nanoparticules d'argent exprimés en masse par rapport à la masse totale de nanoparticules à 95% de particules d'or et 5% de nanoparticules d'argent exprimés en masse par rapport à la masse de nanoparticules. Dans un mode de réalisation avantageux de l'invention, le mélange contient 50% en masse de nanoparticules d'or et 50% en masse de nanoparticules d'argent exprimés en masse par rapport à la masse totale de nanoparticules.

Dans le kit selon l'invention, la solution ou la suspension comprenant les nanoparticules métalliques non magnétiques peut se présenter dans un récipient qui peut être par exemple un tube à essai muni d'un système de fermeture ou un flacon muni d'un système de fermeture, ou un tube conique muni d'un système de fermeture comme par exemple un tube de type Eppendorf®.

La présente invention a également pour objet l'utilisation d'un kit comme définie dans la revendication 11.

De manière optionnelle, le logiciel peut fournir en outre un diagnostic de la maladie liée à la présence du SARS-Cov-2.

Dans la méthode selon l'invention, le modèle de classification peut comprendre des moyens logiciels par apprentissage automatique ou intelligence artificielle.

Le modèle de classification peut comprendre au moins un parmi : un réseau de neurones, une forêt aléatoire, une machine à vecteur de support, une machine à vecteur de pertinence, une PLSDA, et/ou un modèle bayésien.

Le modèle de classification peut comprendre au moins un parmi : un réseau de neurones et/ou une forêt aléatoire.

La méthode selon l'invention peut comprendre, entre la réception et la reconnaissance, une étape de pré-traitement des signaux de spectroscopie Raman exaltée de surface, comprenant de préférence au moins un des prétraitements suivants : une réduction de moyenne, une variation normale standard, une normalisation par le maximum, une normalisation par les extrema, un lissage de préférence par algorithme de Savitzky-Golay, une réduction ou correction de ligne de base, une dérivation d'ordre 1 ou 2, une analyse en composantes principales (ACP).

La méthode selon l'invention peut comprendre, entre la réception et la reconnaissance, une détermination du modèle de classification utilisé parmi plusieurs modèles de classification en fonction d'une sélection d'une forme de prélèvement de l'échantillon et/ou d'une sélection d'un modèle de spectromètre.

Afin d'associer chaque signal de spectroscopie Raman exaltée de surface reçu à chaque classe représentative d'une présence ou une absence de l'agent pathogène SARS-Cov-2 dans l'échantillon, le modèle de classification est

configuré pour appliquer au moins un traitement relatif aux (c'est-à-dire prenant en compte) quatre pics dans les signaux de spectroscopie Raman exaltée suivants :

- un pic à un décalage Raman compris entre 560 $cm^{-1}$ et 760 $cm^{-1}$,
- un pic à un décalage Raman compris entre 1100 $cm^{-1}$ et 1250
- un pic à un décalage Raman compris entre 1250 $cm^{-1}$ et 1500 $cm^{-1}$, et
- un pic à un décalage Raman compris entre 2062 $cm^{-1}$ et 2162 $cm^{-1}$.

La méthode selon l'invention peut comprendre :

a) après la mise en contact dudit échantillon avec des nanoparticules métalliques non magnétiques pour obtenir une solution ou une suspension :
b) le dépôt de ladite solution ou de ladite suspension sur un support ; et
c) la détection des signaux SERS émis par ledit dépôt.

L'ensemble des étapes a) à c) se fait à température ambiante.

Cette méthode de détection permet de détecter la présence d'une infection due au pathogène et de distinguer dans une population les individus malades (porteurs du pathogène et appelés individus positifs) des individus sains (non porteurs du pathogène et appelés individus négatifs). Lorsque l'individu a une infection mais ne présente pas de symptômes de maladie alors la détection de la présence du pathogène sera prédictive d'un risque de développer la maladie.

La méthode selon l'invention peut être utilisée pour évaluer l'efficacité de médicaments ou de vaccins anti-pathogènes connus ou pour tester l'efficacité de nouveaux médicaments ou vaccins anti-pathogènes potentiels. Une diminution de la quantité de l'agent pathogène ou une augmentation de cette quantité indique que la thérapie est efficace ou non.

Dans un mode de réalisation, l'échantillon peut être mis en contact avec lesdites nanoparticules métalliques non magnétiques. Dans un premier mode de réalisation de la méthode selon l'invention on ajoute un volume d'échantillon à tester d'environ 10 à 200 microlitres à environ 10 à 2000 microlitres d'une solution ou d'une suspension contenant des nanoparticules métalliques non magnétiques ou un mélange de nanoparticules non magnétiques d'un premier métal et de nanoparticules non magnétiques d'un second métal, les nanoparticules du premier métal étant différentes des nanoparticules du second métal. Après homogénéisation par agitation par pipetage successifs (pas besoin de vortex), on réalise un dépôt sur un support en aluminium ou un matériau recouvert d'une feuille d'aluminium. Le support peut être n'importe quel type de support couramment utilisée pour la SERS.

Dans ce mode de réalisation, et de façon optionnelle, l'échantillon est mis simultanément en contact avec lesdites nanoparticules métalliques non magnétiques et avec un tampon de lyse, avant l'homogénéisation et le dépôt décrits ci-dessus.

Dans un autre mode de réalisation, l'échantillon peut être mis en solution dans un liquide, dit « milieu de transport », avant la mise en contact avec un culot de centrifugation (également appelé culot de nanoparticules par la suite) contenant lesdites nanoparticules métalliques non magnétiques.

Par exemple, l'échantillon à tester est mis en solution dans le milieu de transport puis mélangé à un culot de nanoparticules obtenu par centrifugation d'une suspension colloïdale de nanoparticules métalliques non magnétiques telles que définies précédemment. Après homogénéisation par agitation, on réalise un dépôt sur un support en aluminium ou sur un matériau recouvert d'une feuille d'aluminium. Le support peut être n'importe quel type de support couramment utilisée pour la SERS.

Dans ce mode de réalisation, et de façon optionnelle, le milieu de transport est un tampon de lyse.

Pour obtenir le culot de nanoparticules, la suspension de nanoparticules métalliques non magnétiques telle que définie précédemment est centrifugée dans des conditions connues de l'homme du métier par exemple à une vitesse de centrifugation de 700 à 18000 g pendant un temps de centrifugation compris entre 1 et 50 minutes.

De préférence, pour obtenir le culot de nanoparticules, la suspension de nanoparticules métalliques non magnétiques est centrifugée à une vitesse inférieure à 5000 g, avantageusement inférieure à 2500 g, par exemple inférieure ou égale à 1000 g. Dans un exemple, la suspension de nanoparticules métalliques non magnétiques centrifugée à une vitesse égale à 800 g.

Ceci est avantageux dans la mesure où il a été observé qu'une centrifugation à des vitesses inférieures à 5000 g, typiquement inférieures à 1500 g, conduit à une augmentation de l'amplitude du signal SERS. Ceci découle vraisemblablement de contraintes mécaniques moins importantes sur les nanoparticules métalliques non magnétiques, qui

subiraient alors une moindre détérioration durant la centrifugation.

Bien évidemment, afin d'obtenir une concentration attendue de nanoparticules métalliques non magnétiques dans le culot de centrifugation, le temps de centrifugation est adapté en fonction de la vitesse de centrifugation. Plus précisément, pour une même suspension centrifugée, et pour une concentration attendue donnée de nanoparticules métalliques non magnétiques dans le culot de centrifugation, le temps de centrifugation augmente généralement quand la vitesse de centrifugation diminue.

Par exemple, une concentration souhaitée de nanoparticules métalliques non magnétiques dans le culot de centrifugation est comprise entre 5 g/L et 30 g/L.

Dans le cas où la suspension de nanoparticules métalliques non magnétiques est centrifugée à une vitesse inférieure à 5000 g, avantageusement inférieure à 2500 g, par exemple inférieure ou égale à 1000 g, le temps de centrifugation est, par exemple, inférieur à une heure.

Dans un exemple, la suspension de nanoparticules métalliques non magnétiques centrifugée à une vitesse égale à 800 g pendant 45 min.

Conformément à l'invention, le dépôt est susceptible d'être séché, dans des conditions classiques connues de l'homme du métier.

Les nanoparticules métalliques non magnétiques peuvent présenter un diamètre compris entre 50 et 200 nm, avantageusement compris entre 100 et 200 nm, encore plus avantageusement compris entre 100 et 150 nm.

Les nanoparticules métalliques non magnétiques du premier métal peuvent être des particules d'or et les nanoparticules métalliques non magnétiques du second métal peuvent être des nanoparticules d'argent.

La réception de signaux de spectroscopie Raman exaltée de surface peut comprendre :

- une émission de lumière d'excitation, de préférence de longueur d'onde comprise entre 750 et 800 nm, ladite lumière d'excitation atteignant l'échantillon,
- une captation, par un capteur ou spectromètre, de lumière réfléchie, transmise, diffusée ou rétrodiffusée par l'échantillon alors que ladite lumière d'excitation atteint l'échantillon.

La lumière d'excitation peut atteindre l'échantillon et le spectromètre et/ou le capteur peut mettre en œuvre l'étape de captation alors que l'échantillon a été mis en contact avec des nanoparticules.

Conformément à l'invention, tout système de spectromètre Raman approprié connu dans la technique et disponible dans le commerce peut être utilisé.

Les dispositifs de détection, tels que les détecteurs optiques, les sources de rayonnement et les systèmes informatiques, les microprocesseurs et les logiciels et algorithmes informatiques, peuvent être utilisés dans n'importe quelle combinaison pour mettre en pratique la méthode selon l'invention. En conséquence, dans certains modes de réalisation, un logiciel ou d'autres instructions lisibles par ordinateur peuvent être utilisés pour interpréter, analyser, compiler ou autrement analyser des données de sortie. Le logiciel ou un autre système informatique peut être utilisé pour afficher, stocker ou transmettre des données de sortie, que ce soit sous forme numérique ou autre, à un ou plusieurs utilisateurs.

Pour un pathogène donné, la sélection des longueurs d'onde est réalisée par toute technique connue de l'homme du métier ou décrite dans la littérature, notamment par le biais d'un algorithme tel décrit par Marois M. et al., ou par Chen Y. et al., ou par Luke G.P. et al.

Cette méthode permet de mesurer les spectres SERS de différents pathogènes. Chaque pathogène peut être détecté parce qu'il possède un spectre SERS unique qui est sensiblement différent, et donc distinguable, des spectres SERS d'autres pathogènes. Ainsi les pathogènes, notamment les virus, ont une « signature » SERS unique qui permet de distinguer une biomolécule d'intérêt ou une combinaison de biomolécules particulières des autres biomolécules ou des milieux de base.

Typiquement en présence de pathogène inactivé ou en l'absence de pathogène, on obtient une première signature spectroscopique Raman exaltée de surface et lorsque le pathogène est présent on obtient une seconde signature spectroscopique Raman exaltée de surface différente de la première.

L'invention concerne aussi des moyens logiciels, conçus et/ou agencés et/ou programmés pour mettre en œuvre une méthode selon l'invention.

L'invention concerne aussi un système, conçu et/ou agencé et/ou programmé pour mettre en œuvre une méthode selon l'invention.

Les inventeurs ont trouvé que la présence du SARS-CoV-2 dans un échantillon pour des nanoparticules d'or est caractérisée par la présence d'un pic entre 560 $cm^{-1}$ et 760 $cm^{-1}$ (typiquement à 660 ou 727 $cm^{-1}$, de préférence 660 $cm^{-1}$), d'un pic entre 1250 et 1500 $cm^{-1}$ (typiquement à 1374 $cm^{-1}$) et d'un pic entre 2062 $cm^{-1}$ et 2162 $cm^{-1}$ (typiquement à 2100 ou 2112 $cm^{-1}$, de préférence 2100 $cm^{-1}$). On dit alors que le patient chez lequel l'échantillon a été prélevé est positif SARS-CoV-2. En revanche en l'absence de virus dans un échantillon, seul un pic entre 1100 et 1250 nm est visible. Le patient est alors déclaré négatif au SARS-CoV-2.

L'invention a également pour objet l'utilisation d'un kit selon la présente invention dans lequel le logiciel fournit en outre un diagnostic de la maladie liée à la présence dudit agent pathogène.

**Description des figures et modes de réalisation**

D'autres avantages et particularités de l'invention apparaîtront à la lecture de la description détaillée de mises en œuvre et de modes de réalisation nullement limitatifs, et des dessins annexés suivants :

la **figure 1** illustre les résultats obtenus avec des prélèvements naso-pharyngés de 20 personnes différentes avec 3 échantillons pour chaque prélèvement. Les conditions expérimentales sont celles de l'exemple 1 de l'invention
la **figure 2** donne les spectres obtenus dans les conditions de l'exemple 1 pour des patients positifs chez lesquels on a détecté la présence de Covid-19 (en noir) et chez des patients négatifs chez lesquels on n'a pas détecté la présence du Covid-19 (en gris)
la **figure 3** illustre la présence des pics chez des patients positifs (+) et chez des patients négatifs (x) lorsqu'on utilise des nanoparticules d'or selon l'invention
la **figure 4** est une vue schématique d'un premier mode de réalisation de système 10 selon l'invention
la **figure 5** présente une série de mesures (signaux de spectroscopie Raman exaltée de surface (SERS)) réalisées avec le spectromètre STRam pour un patient
la **figure 6** présente une série de mesures (signaux de spectroscopie Raman exaltée de surface (SERS)) avant et après prétraitement par SNV (Série de mesure avant (A) et après (B) prétraitement par SNV, données mesurées par spectromètre STRam)
la **figure 7** illustre un exemple d'arbre de décision, volontairement laissé de petite taille, pour la classification de spectres utilisé dans le cadre de la présente invention
la **figure 8** illustre schématiquement un réseau de neurones utilisé dans le cadre de la présente invention
la **figure 9** illustre l'aspect des spectres MIRASA après prétraitement,
la **figure 10** présente l'aspect des spectres STRam après prétraitement complet
la **figure 11** illustre la matrice de confusion obtenue en validation pour le modèle STRam
la **figure 12** illustre la matrice de confusion pour la prédiction par patient du modèle STRam en validation
la **figure 13** illustre la matrice de confusion obtenue en validation pour le modèle MIRASA
la **figure 14** illustre la matrice de confusion pour la prédiction par patient du modèle MIRASA en validation
la **figure 15** illustre la répartition des pics de décalage Raman pour 2196 échantillons positifs au SARS-Cov-2.

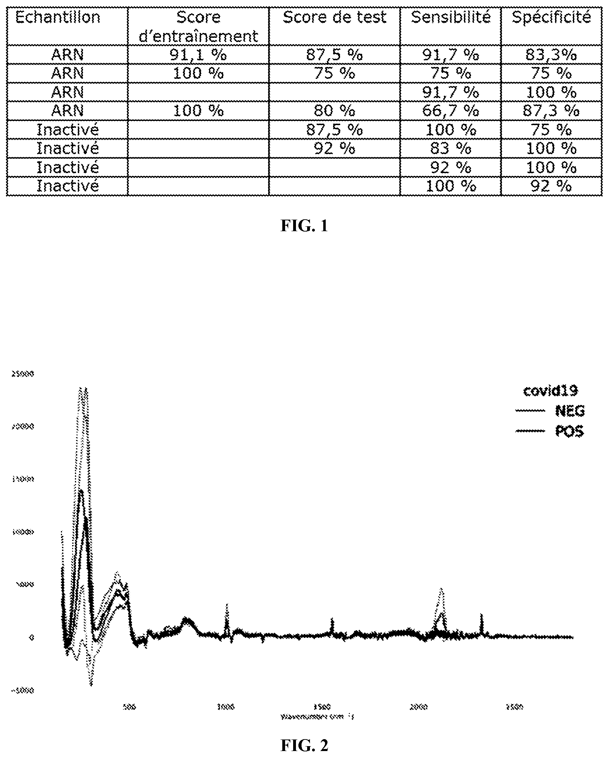

Sur l'ensemble des spectres des figures 2, 3, 5, 6, 9 et 10 :

- l'abscisse est le décalage Raman en $cm^{-1}$, et
- l'ordonnée est une intensité en unité arbitraire.

Ces modes de réalisation n'étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

Dans un premier mode de réalisation nullement limitatif, un kit selon l'invention pour la détection de la présence d'un agent pathogène dans un échantillon par spectroscopie Raman exaltée de surface (SERS) comprend des nanoparticules métalliques non magnétiques et un logiciel et/ou des moyens logiciels conçu(s) et/ou agencé(s) et/ou programmé(s) pour détecter la présence dudit agent pathogène dans ledit échantillon.

De façon très avantageuse, dans tous les exemples décrits, et dans toutes les variantes envisagées, les nanoparticules métalliques non magnétiques sont des nanoparticules métalliques natives non magnétiques.

De façon optionnelle, le kit selon l'invention comprend également un tampon de lyse.

Les fonctions techniques de ce logiciel ou de ces moyens logiciels seront décrites plus en détails par la suite.

L'agent pathogène qui est détecté est le SARS-CoV-2.

De préférence, les nanoparticules métalliques non magnétiques présentent un diamètre moyen compris entre 50 et 200 nm, avantageusement compri s entre 100 et 200 nm, encore plus avantageusement compris entre 100 et 150 nm.

De préférence, les nanoparticules métalliques non magnétiques sont des particules d'or, d'argent, de cuivre, de

platine ou d'un alliage à base d'un de ces métaux. En particulier, l'or, l'argent et/ou le platine sont utilisés car ces métaux n'altèrent pas les prélèvements biologiques.

Par exemple, les nanoparticules métalliques non magnétiques comprennent un mélange de nanoparticules non magnétiques d'un premier métal et de nanoparticules non magnétiques d'un second métal, les nanoparticules du premier métal étant différentes des nanoparticules du second métal.

Dans ce cas, et de façon préférée, les nanoparticules métalliques non magnétiques du premier métal sont des nanoparticules d'or et les nanoparticules métalliques non magnétiques du second métal sont des nanoparticules d'argent.

On utilise ce kit comprenant des nanoparticules métalliques non magnétiques et un logiciel conçu pour détecter la présence d'un agent pathogène dans un échantillon par spectroscopie Raman exaltée de surface (SERS). Comme indiqué précédemment, le kit comprend, de façon optionnelle, un tampon de lyse.

Le logiciel ou les moyens logiciels du kit peu(ven)t en outre, mais de manière optionnelle, fournir un diagnostic de la maladie liée à la présence dudit agent pathogène.

Un premier mode de réalisation nullement limitatif de système 10 selon l'invention comprend :

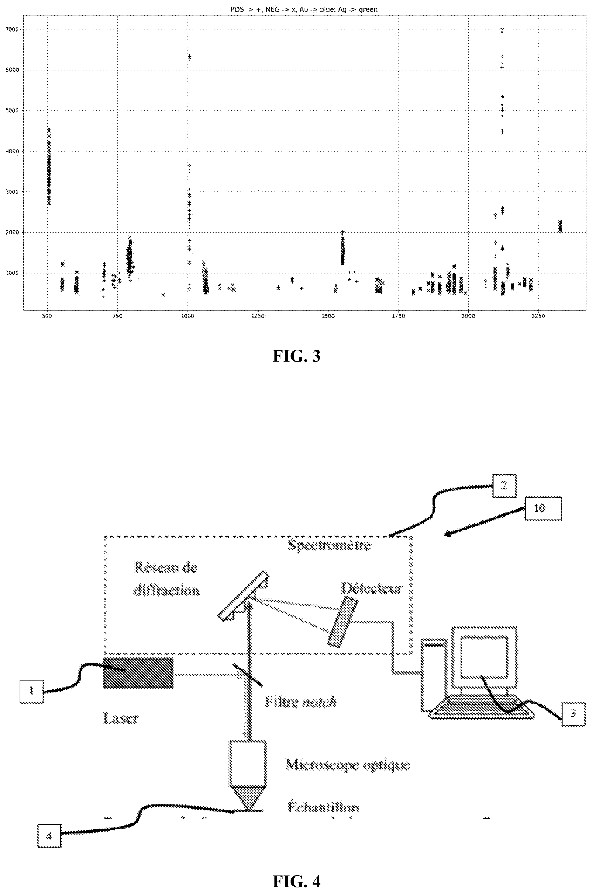

- un dispositif optique tel qu'illustré sur la figure 4 , comprenant :
    - ◦ un porte échantillon 4,
    - ◦ une source 1 (typiquement un laser) d'émission de lumière d'excitation, ladite lumière comprenant au moins une longueur d'onde comprise entre 750 et 800 nm (par exemple un longueur d'onde à 785 nm), ladite source 1 étant agencée pour que ladite lumière d'excitation atteigne un échantillon sur le porte échantillon,
    - ◦ un capteur ou spectromètre 2 (typiquement un spectrophotomètre Raman), et agencé pour capter de la lumière réfléchie, transmise, diffusée ou rétrodiffusée par l'échantillon alors que ladite lumière d'excitation atteint l'échantillon ; l'élément 2 comprend typiquement un réseau de diffraction agencé pour diffracter la lumière réfléchie, transmise, diffusée ou rétrodiffusée par l'échantillon et un détecteur agencé pour détecter la lumière ainsi diffractée,
- le logiciel ou les moyens logiciel du kit,
- une unité d'analyse 3, comprenant au moins un ordinateur, une unité centrale ou de calcul, un circuit électronique analogique (de préférence dédié), un circuit électronique numérique (de préférence dédié), et/ou un microprocesseur (de préférence dédié), et agencé et/ou programmé pour mettre en œuvre le logiciel ou les moyens logiciel du kit.

Dans le cas où la source 1 est un laser, ladite source 1 est, par exemple, configurée pour délivrer un faisceau laser présentant une puissance comprise entre 100 mW et 1 W, par exemple 500 mW. Dans ce dernier cas, le dépôt est éclairé pendant quelques secondes, typiquement entre 0,1 s et 20 s, par exemple entre 1 s et 7 s.

Le premier mode de réalisation de méthode d'utilisation du kit et/ou de détection d'un agent pathogène dans des données de spectroscopie Raman exaltée de surface (SERS), mis en œuvre dans le système 10, va maintenant être décrit.

Dans ce premier mode de réalisation de méthode selon l'invention, l'unité 3 réceptionne des signaux de spectroscopie Raman exaltée de surface générés de la manière suivante :

a) on met en contact un échantillon (typiquement un prélèvement biologique tel que de préférence un prélèvement salivaire ou nasopharyngé d'un homme ou d'un animal) avec les nanoparticules métalliques non magnétiques pour obtenir une solution ou une suspension ; puis

b) on dépose ladite solution ou ladite suspension sur un support, plus précisément sur le porte échantillon 4 et

c) on détecte des signaux SERS émis par ledit agent pathogène, les signaux indiquant la présence dudit agent pathogène, de préférence par :

- une émission (par la source 1) de lumière d'excitation, de préférence de longueur d'onde comprise entre 750 et 800 nm, ladite lumière d'excitation atteignant l'échantillon ; ladite lumière d'excitation atteint l'échantillon alors que l'échantillon est en contact avec les nanoparticules,
- une captation, par le capteur ou spectromètre 2, de lumière réfléchie, transmise, diffusée ou rétrodiffusée par l'échantillon alors que ladite lumière d'excitation atteint l'échantillon ; le spectromètre ou capteur 2 met en œuvre l'étape de captation alors que l'échantillon est en contact avec les nanoparticules.

Comme indiqué précédemment, les nanoparticules métalliques non magnétiques comprennent, par exemple, un mélange de nanoparticules non magnétiques d'un premier métal et de nanoparticules non magnétiques d'un second métal, les nanoparticules du premier métal étant différentes des nanoparticules du second métal. Dans ce cas, les nanoparticules métalliques non magnétiques du premier métal sont, par exemple, des particules d'or et les nanoparticules

métalliques non magnétiques du second métal sont, par exemple, des nanoparticules d'argent.

De préférence, les nanoparticules métalliques non magnétiques présentent un diamètre compris entre 50 et 200 nm, avantageusement compris entre 100 et 200 nm, encore plus avantageusement compris entre 100 et 150 nm.

Selon la variante considérée :

- l'échantillon est mis en solution dans un milieu de transport avant la mise en contact avec un culot de centrifugation contenant lesdites nanoparticules métalliques non magnétiques, ou
- l'échantillon est mis simultanément en contact avec lesdites nanoparticules métalliques non magnétiques.

Dans chaque variante, les nanoparticules métalliques non magnétiques sont, notamment en suspension colloïdale, par exemple dans du citrate de sodium.

Alternativement :

- l'échantillon est mis en solution dans un tampon de lyse avant la mise en contact avec le culot de centrifugation, ou
- l'échantillon est mis simultanément en contact avec un tampon de lyse et avec des nanoparticules métalliques non magnétiques.

Ensuite, l'unité 3 effectue les étapes suivantes :

- une réception, par l'unité 3, de signaux de spectroscopie Raman exaltée de surface (SERS) en provenance du capteur 2 ou spectromètre 2 et obtenus à partir de l'échantillon,
- une reconnaissance, par un modèle de classification (faisant partie du logiciel ou des moyens logiciels du kit), des signaux de spectroscopie Raman exaltée comme étant des signaux indiquant une présence ou une absence de l'agent pathogène dans l'échantillon.

Dans la présente description, on utilisera indifféremment les expressions « signaux de spectroscopie Raman exaltée de surface (SERS) » ou « données de spectroscopie Raman exaltée de surface (SERS) ».

Les signaux de spectroscopie Raman exaltée de surface (SERS) comprennent des données de décalage Raman comprises au moins entre 500 $cm^{-1}$ et 2300 $cm^{-1}$.

Le spectromètre 2 est par exemple :

- un spectromètre STRam fournissant des données de décalage Raman comprises entre 499,46 et 2801,89 $cm^{-1}$ ; ou
- un spectromètre MIRA fournissant des données de décalage Raman comprises entre 499 et 2300 $cm^{-1}$.

Le modèle de classification comprend les moyens logiciels du kit qui sont des moyens logiciels par apprentissage automatique (ou *« machine learning »* en anglais) ou intelligence artificielle.

Le modèle de classification comprend au moins un parmi : un réseau de neurones, une forêt aléatoire, une machine à vecteur de support, une machine à vecteur de pertinence, un PLS-DA (ou *« Partial least squares discriminant analysis »* en anglais ou « Analyse des moindres carrés partiels » en français), et/ou un modèle bayésien. De préférence, le modèle de classification comprend au moins un parmi : un réseau de neurones et/ou une forêt aléatoire.

De façon optionnelle, la méthode comprend, entre la réception et la reconnaissance, une étape de prétraitement des signaux de spectroscopie Raman exaltée, comprenant de préférence au moins un des prétraitements suivants : une réduction de moyenne, une variation normale standard (ou *« Standard Normal Variate »* (SNV) en anglais), une normalisation par le maximum, une normalisation par les extrema, un lissage de préférence par algorithme de Savitzky-Golay, une réduction ou correction de ligne de base, une dérivation de préférence d'ordre 2, une analyse en composantes principales ACP (ou PCA, de l'anglais *« Principal Component Analysis »*).

La méthode comprend, entre la réception et la reconnaissance, une détermination du modèle de classification utilisé parmi plusieurs modèles de classification prédéterminés en fonction :

- d'une forme de prélèvement de l'échantillon (typiquement, un utilisateur rentre sur un clavier ou un écran tactile de l'unité 3 la forme du prélèvement testé, typiquement nasopharyngé ou salivaire) ; et/ou
- d'un modèle de spectromètre 2 (typiquement, un choix par défaut est programmé et/ou un utilisateur rentre sur un clavier ou un écran tactile de l'unité 3 le modèle de spectromètre 2 (typiquement STRam ou MIRA) utilisé dans le système 10) ; et/ou
- d'un milieu de transport de l'échantillon ; et/ou

- des données relatives au sujet sur lequel a été prélevé l'échantillon, telles que la symptomatologie, des résultats d'examens complémentaires (par exemple, des résultats d'imagerie médicale), l'âge, le sexe ; et/ou
- de l'agent ou des agents pathogène(s) à détecter.

Un tel choix est effectué, de préférence, de façon automatique.
L'agent pathogène que l'on cherche à détecter est le SARS-CoV-2 (coronavirus 2 du syndrome respiratoire aigu sévère).
Les inventeurs ont constaté que des pics à 439, 586, 666, 739, 859, 982, 1026, 1141, 1210, 1359, 1549, 1611, 1682, 1742, 1816, 2078, 2130, 2342 et/ou 2480 $cm^{-1}$ étaient particulièrement discriminants pour la détection du SARS-CoV-2 dans un échantillon.

**Base de données**

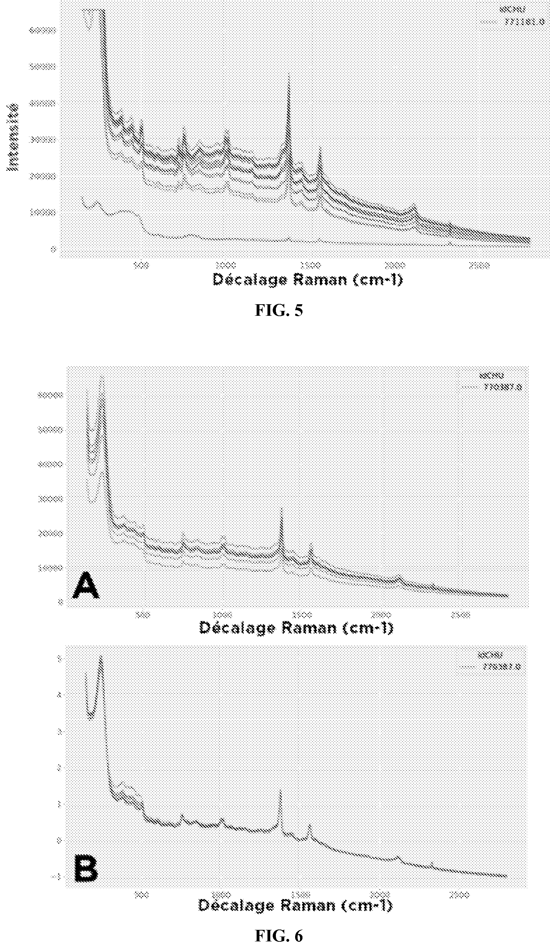

L'apprentissage automatique, par le modèle de classification, s'est construit sur une base de données.
Par exemple, pour le modèle de classification spécifique à la forme de prélèvement nasopharyngé, la base de données est constituée de 110 échantillons (i.e. patients) : 55 prélèvements nasopharyngés de patients déclarés positifs (POS) au COVID-19 et 55 prélèvements nasopharyngés de patients déclarés négatifs (NEG) au COVID-19. Le test de dépistage du virus SARS-CoV-2 a été réalisé par la méthode RT-PCR (« *Reverse Transcription-Polymerase Chain Reaction* » ou transcription inverse - Réaction en chaîne par polymérase en français). Les prélèvements et les tests ont été réalisés au CHU d'Amiens.
Chaque échantillon a fait l'objet d'une préparation avec des nanoparticules tel que précédemment décrit. Cette préparation est ensuite déposée sur trois lames distinctes (appelés dépôts par la suite). Chaque dépôt est analysé trois fois par spectroscopie Raman dans le système 10 engendrant donc un total de 9 spectres par échantillon. Un ensemble de spectres relatifs à un patient est appelé une série. De plus, 8 patients positifs et 8 patients négatifs ont fait l'objet de mesures répétées sur une autre journée d'analyse afin de contrôler un éventuel impact des conditions d'expérimentation sur la mesure Raman. Ces données ont été incluses dans la base de données, engendrant donc un total de 567 spectres étiquetés « POSITIFS » (abrégé POS par la suite) et 567 spectres étiquetés « NEGATIFS » (abrégé NEG par la suite).
Puisqu'il s'agit de données issues d'un protocole en laboratoire, il est possible que des facteurs expérimentaux aient un impact sur les spectres (ex : préparation ou dépôt mal réalisé(e), analyse spectrale sur le bord du dépôt, mauvaise référence du spectromètre...). Ces aléas peuvent engendrer une allure spectrale différente de celle attendue, auquel cas on parlera de spectre « outlier », c'est-à-dire de spectres « aberrants ». Plusieurs méthodes peuvent être employées pour travailler avec ces spectres : il convient d'abord de les identifier puis de les mettre à l'écart. On peut alors choisir d'entraîner un modèle uniquement sur les spectres non outliers ou de les intégrer dans la base afin d'entrainer un modèle prédictif à les identifier (on peut alors imaginer une alerte logicielle précisant qu'une mesure est improprement réalisée et qu'elle ne sera pas prise en compte). Puisque le résultat final est accompagné d'une probabilité d'appartenance à la classe directement influencé par l'allure du spectre, les outliers sont supprimés de la base de données. La figure 5 présente une série de mesures réalisées avec le spectromètre STRam pour un patient 771181 où sont présents des spectres outliers.
Sur ces spectres, on note deux phénomènes. Premièrement, deux spectres ont une allure radicalement différente du reste de la série de mesure. Il s'agit de deux spectres présentant une faible intensité et traduisant donc une acquisition ne visant pas correctement le dépôt réalisé sur la lame. Ces deux spectres ont donc été supprimés de la base de données. Deuxièmement, on observe un phénomène de saturation du signal en début de gamme. Ce phénomène, se produisant sur une bonne partie de la base de données, a engendré un choix particulier pour les prétraitements décrits par la suite.
L'exemple de la figure 5 présente un cas particulier d'outliers pour un échantillon particulier (patient 771181), l'analyse effectuée sur les 110 patients a engendré un filtrage conséquent de la base de données détaillé dans le tableau 1, qui illustre le nombre de spectres présents en base après filtrage pour les spectromètres STRam et MIRA.

| Spectromètre | Label | Nombre de spectres initial | Nombre de spectres final |
|---|---|---|---|
| STRam | POSITIF (POS) | 567 | 447 |
| | NEGATIF (NEG) | 567 | 504 |
| MIRA | POSITIF (POS) | 567 | 516 |
| | NEGATIF (NEG) | 567 | 507 |

Tableau 1

Ces bases de données filtrées sont celles qui ont servi de jeux d'entrainement et de validation des modèles prédictifs de classification de l'unité 3 décrits dans la prochaine partie.

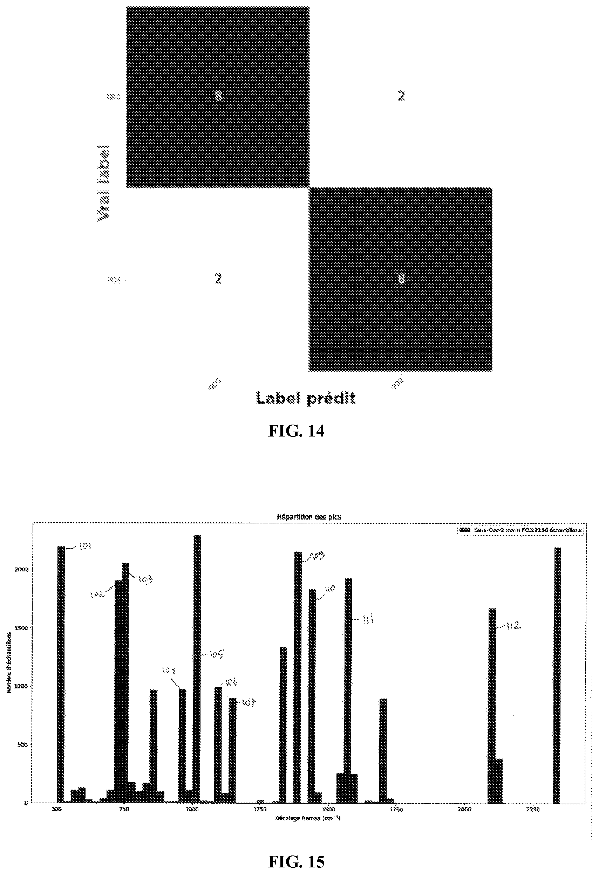

La figure 15 illustre la répartition des pics de décalage Raman pour 2196 échantillons positifs au SARS-Cov-2. Les pics 101 à 107 et 109 à 112, dont la présence est indicatrice de la présence du SARS-CoV-2, sont visibles sur cette figure 15.

**Modèles de classification de l'unité 3**

Lorsque l'on travaille avec un classifieur binaire, l'outil de base afin d'analyser les performances de prédiction est la matrice de confusion. Il s'agit d'un tableau à double entrée 2x2 confrontant les labels prédits aux labels réels, comme illustré dans le tableau 2.

Tableau 2

| Label réel | NEG | VN | FP |
|---|---|---|---|
| | POS | FN | VP |
| | | NEG | POS |
| | | Label prédit | |

Ce genre de matrice met directement en évidence les forces et les faiblesses du classifieur. En effet, on cherche à maximiser le nombre de vrais négatifs (VN) et vrais positifs (VP), qui correspondent à des prédictions correctes, tout en minimisant le nombre de faux négatifs (FN) et faux positifs (FP), qui correspondent à des erreurs. On peut d'ailleurs calculer des métriques directement à partir de ces grandeurs :

$$Précision = \frac{VP + VN}{VP + FP + VN + FN}$$

$$Sensibilité = \frac{VP}{VP + FN}$$

$$Spécificité = \frac{VN}{VN + FP}$$

Ces trois métriques sont des grandeurs importantes quand on cherche à quantifier les performances d'un classifieur binaire. La précision correspond au pourcentage d'éléments correctement prédits. La sensibilité correspond à la probabilité que le classifieur renvoie « POSITIF » si la maladie est réellement présente. Pareillement, la spécificité correspond à la probabilité que le classifieur renvoie « NEGATIF » pour un patient non malade. Lorsque l'on travaille avec un classifieur binaire, une valeur de précision, sensibilité ou spécificité proche de 0,5 signifie qu'on effectue une aussi

bonne prédiction qu'un choix aléatoire tandis que se rapprocher de 1,0 montre un bon pouvoir prédictif.

Maximiser la précision est forcément une bonne chose. En effet, plus celle-ci est proche de 1,0 plus le pourcentage de spectres correctement classés est élevé. Cependant, dans le cadre d'un test de dépistage de maladie, on se contentera souvent de maximiser la spécificité du modèle (tout en conservant une sensibilité la plus élevée possible). En effet, on préfèrera avoir la certitude qu'un test déclarant « négatif » ne se trompe pas tandis qu'on pourra confirmer un test déclarant « positif » en répétant le test par exemple. Dans un contexte de pandémie, tel qu'il se présente pour le SARS-CoV-2, l'approche doit être différente. En effet, il est crucial de bien détecter si un patient est atteint de la maladie afin d'empêcher que celui-ci ne contamine d'autres personnes et déclarer un patient comme étant positif s'il ne l'est pas peut être considéré comme un risque acceptable. Dans cette optique, le choix des meilleures modélisations se fait principalement sur le critère de la sensibilité.

L'optimisation de modèles prédictifs est une tâche coûteuse en ressource de calcul, à la fois dépendante du nombre et de la dimension des données. Puisqu'il est nécessaire de comparer les performances de plusieurs modèles, qui doivent être optimisés au préalable, la réduction de la dimension des données est un prétraitement qui peut grandement améliorer les temps de calcul. Pour cette raison, les données du spectromètre 2 MIRA et du STRam ont été transformées par Analyse en Composante Principale (ACP). Cette méthode diagonalise la matrice de covariance d'un jeu de données afin d'en extraire les vecteurs propres. Ces vecteurs, également appelés composantes principales, servent ensuite de nouvelle base sur laquelle projeter les données. L'ACP permet de réduire grandement la dimension des données tout en conservant la partie pertinente de l'information présente dans le jeu initial. A titre d'exemple, les 951 spectres sortant du STRam possèdent 1959 points mais une ACP permet, en utilisant 12 composantes, de conserver plus de 99.9% de la variabilité interne aux données. On passe alors d'une matrice de taille 951x1959 à une matrice de taille 951x12 en conservant la quasi-totalité de l'information. Certains modèles, sensibles à la dimensionnalité des données, sont optimisés en un temps 100 fois inférieur en procédant de la sorte.

Avant de réduire la dimension des données, les spectres ont été prétraités par SNV *(Standard Normal Variate* ou « variation normale standard » en français). Il s'agit d'un prétraitement commun dans le milieu de la chimiométrie qui consiste à soustraire à chaque spectre sa moyenne (centrage) et de le diviser par son écart-type. Après un prétraitement par SNV, un spectre présente une moyenne nulle et un écart type unitaire. La figure 6 présente une série de mesure avant et après prétraitement par SNV (Série de mesure avant (A) et après (B) prétraitement par SNV, données mesurées par le STRam). Un tel prétraitement est optionnel.

L'intensité acquise pour des spectres dépend du chemin optique parcouru par la lumière au cours de la mesure, qui dépend lui-même de la nature de l'échantillon. Ainsi, en fonction de la matrice que l'on scanne il peut être difficile d'avoir des conditions de mesure parfaitement reproductibles. La SNV permet de réduire très fortement les variations d'intensité générale des spectres et permet de resserrer les séries de mesure, comme on peut le voir entre les spectres du panneau A et du panneau B de la figure 6.

Il existe une grande variété de modèles de classification. Dans le cadre des modes de réalisation nullement limitatifs de la présente invention, plusieurs classifieurs programmés en Python ont été entrainés : des réseaux de neurones, forêts aléatoires, machines à vecteur de support, machines à vecteur de pertinence, PLSDA et modèles bayésiens.

Afin de mesurer les performances des différentes modélisations, on utilise le principe de la validation croisée (CV). Cette méthode divise la base de données d'entraînement en K parties (ou « groupes »), on parle alors de validation croisée K-fold. Dans les modes de réalisation décrits ici, nous avons choisi K = 10. Chacune des 10 parties de la base de données contient sensiblement la même distribution de spectres positifs et négatifs que la base de données initiales, on parle donc de validation croisée stratifiée. De plus, ces groupes ne sont pas découpés aléatoirement. En effet, les 9 spectres d'un patient sont nécessairement présents dans le même groupe afin d'éviter tout biais dans les performances.

Une fois la base de données découpée en 10 parties, un modèle est entrainé successivement sur 9 d'entre elles et testé sur la dernière. Finalement, le modèle est entrainé 10 fois et testé sur tous les différents morceaux de la base, c'est-à-dire les différentes parties de la base. En moyennant les 10 performances du modèle obtenues, c'est-à-dire les performances du modèle sur chacune des 10 parties de la base, on obtient la performance du modèle en validation croisée. Dans ce cas, pour chaque modèle, la performance correspondante est la précision (définie précédemment) obtenue au moyen dudit modèle.

En *machine learning,* on parle d'hyper-paramètre lorsqu'un paramètre d'un modèle est réglé par l'utilisateur et pas durant l'apprentissage. Les autres paramètres sont dits « paramètres prédictifs ».

La quasi-totalité des modèles ont des hyper-paramètres. Par exemple, un classifieur basé sur les forêts aléatoires en possède jusqu'à 17. Certains présentent moins d'intérêt que d'autres mais il est nécessaire de correctement régler ces hyper-paramètres pour obtenir la meilleure modélisation possible. Dans cette optique, les hyper-paramètres de tous les modèles qui ont été testés ont été sélectionnés en utilisant une analyse exhaustive, qu'on appelle « GridSearch ». Cette approche utilise un estimateur (par exemple une forêt aléatoire) et un espace des hyper-paramètres qui doivent être testés. Toutes les combinaisons possibles dans l'espace des hyper-paramètres fournis sont alors testées et sont associées à un score de validation croisée.

Lorsqu'un modèle possède un grand nombre d'hyper-paramètres que l'on cherche à optimiser, la GridSearch peut vite engendrer un grand nombre d'estimateurs (c'est-à-dire de paramètres prédictifs) à optimiser. En effet, lorsque les modèles présentent un grand nombre d'hyper-paramètres, il est possible que les paramètres optimaux différent d'un espace d'hyper-paramètres à un autre. Dans ce cas, tous les espaces d'hyper-paramètres sont testés, et des modèles sont construits avec toutes les combinaisons possibles de paramètres ; enfin le modèle avec les performances les plus élevées (et donc les paramètres prédictifs et les hyper-paramètres associés à ce modèle) est retenu.

Le tableau 3 présente un des espaces d'hyper-paramètres envisageable pour une forêt aléatoire (ces paramètres seront détaillés plus en profondeur par la suite) :

Tableau 3

| | |
|---|---|
| Nombre d'estimateurs (i.e. le nombre d'arbres présents dans la forêt) « N estimators » en anglais | 100, 200, 350, 500, 800 |
| Nombre max de caractéristiques (i.e. le nombre de caractéristiques (ici, des décalages Raman) à prendre en compte pour juger de la qualité d'un nœud de l'arbre. Si on choisit, par exemple, racine carrée, cette valeur sera la racine carrée du nombre total de décalages Raman dans les données.) « Max. features » en anglais | Auto, racine carrée (sqrt), log2 |
| Profondeur maximale (i.e. le nombre « d'étages » de l'arbre. Si elle est fixée à 10, alors les feuilles finales de l'arbres seront atteintes après 10 nœuds) « Max. depth » en anglais | 2, 3, 4, 5, 6, 7, 8, 9, 10, 11, 12, 13, 14, 15 |
| Critère (i.e. le critère utilisé pour juger de la qualité d'un nœud) « Criterion » en anglais | Entropie de Shannon, Coeff. Gini |
| Bootstrap (bootstrap : il s'agit d'une méthode de randomisation de la base de données d'entrainement pour entrainer tous les arbres de la forêt sur des données légèrement différentes.) | Oui, Non |

Un tel espace des hyper-paramètres engendre 840 combinaisons différentes de paramètres. De plus, chaque modèle est entrainé en validation croisée K-fold avec K = 10, ce qui fait un total de 8400 modèles à entrainer et peut représenter plusieurs heures de calcul. On peut ensuite sélectionner le score de validation croisée le plus élevé parmi les 8400 scores calculés et donc retrouver le paramétrage le plus optimisé.

Pour chaque type de modèle on a alors le meilleur paramétrage et un score de validation croisée associé. On est alors en mesure de sélectionner le modèle donnant les meilleurs résultats sur les données du STRam et les données MIRASA (les données MIRASA étant les données obtenues au moyen d'un spectromètre MIRA). La meilleure modélisation obtenue pour les données STRam est une forêt aléatoire, celle obtenue pour les données MIRASA est un réseau de neurones (Perceptron multi-couche).

Forêt aléatoire

Avant de décrire le fonctionnement d'une forêt aléatoire, il est important de comprendre ce qu'est un arbre de décision. Les arbres de décision sont des modèles prédictifs qui peuvent être utilisés en classification comme en régression. C'est le premier de ces cas qui nous intéresse ici.

Un arbre de décision correspond à un organigramme qui va tester les valeurs des différents paramètres prédictifs et avancer dans le graphe en fonction des tests qu'il réalise.

L'entrainement d'un tel modèle consiste à trouver, à partir des données et des labels de la base d'entraînement (c'est-à-dire les labels réels), les différents tests sur les variables prédictives que doit réaliser cet arbre, appelés des « nœuds ». Lorsque tous les nœuds ont été franchis, on arrive à une décision finale (l'attribution du label « POSITIF » ou « NEGATIF » dans notre cas) appelée une « feuille ».

Le test à réaliser pour un nœud fixé est identifié en fonction du critère utilisé pour l'arbre. Pour un arbre de classification ce critère peut être l'entropie de Shannon ou l'indice de diversité de Gini. Dans nos modélisations, les deux critères ont été testés mais c'est l'indice de Gini qui a été conservé car il donnait les meilleurs résultats. Cet indice se calcule à partir de la distribution des données et peut être calculé pour chacune des variables explicatives du jeu de données (à savoir, dans ce cas, la valeur du spectre pour chaque décalage Raman, ou encore la valeur de chaque composante principale si une ACP est mise en œuvre). Pour un nœud donné, plus cet indice est proche de 0, plus ce nœud est dit « pur », c'est-à-dire qu'il met en avant un critère permettant une bonne discrimination. De plus, en testant toutes les variables explicatives en chaque nœud, on trouve normalement pour chaque étape la variable explicative permettant de

donner la meilleure discrimination entre les variables explicatives restantes dans la branche de l'arbre en cours. L'optimisation d'un arbre de décision consiste donc à trouver une succession de nœuds donnant un indice de Gini le plus bas possible pour permettre la meilleure classification globale.

Par $X_N$, on entend le N$^{ème}$ décalage Raman. Il ne s'agit pas de la valeur à N $cm^{-1}$, mais bien de la N$^{ème}$ valeur de la liste de données d'un spectre Raman mémorisé dans l'unité 3.

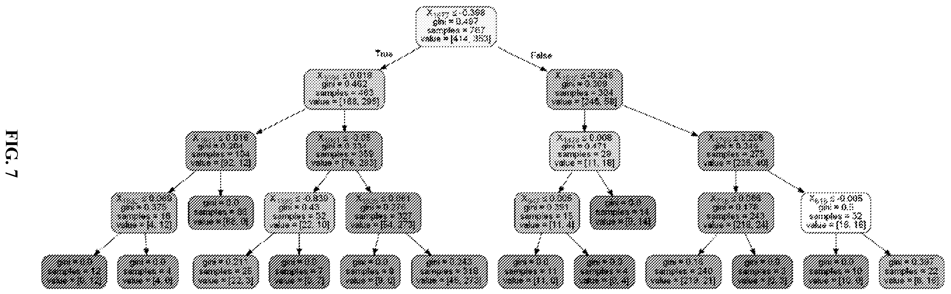

La figure 7 donne un exemple d'un arbre de décision, volontairement laissé de petite taille, pour la classification des spectres de notre étude.

Dans les faits, ces types d'arbre peuvent vite devenir très grands. Pour cette représentation, nous avons fixé la profondeur maximale de l'arbre (qui est un hyper-paramètre du modèle) à 5 pour qu'il reste compréhensible. Pour chacun des nœuds, on voit quel test est fait sur quelle variable (ex : $X_{1877} < -0.398$) ainsi que l'indice de Gini qui y est associé. La ligne « samples » déclare le nombre d'échantillons en entrainement qui sont arrivés à ce nœud et la ligne « value » donne respectivement le nombre de patient NEG et POS que cela représente.

Une fois que ces modèles ont été compris, on peut alors envisager d'en utiliser plusieurs conjointement. C'est ce qu'on appelle une forêt aléatoire. On parlera de forêt aléatoire de 300 arbres lorsque l'on utilise 300 arbres différents pour la modélisation. Si ces forêts portent le nom « aléatoire », c'est que chacun des arbres qui la constitue n'est pas entrainé sur la même base de données, ce qui supprimerait l'intérêt d'utiliser différents modèles. En effet, on réalise dans la base de données originale un tirage aléatoire d'autant de jeux de données que l'on souhaite intégrer d'arbre dans notre forêt. Ainsi, on entraine des modèles prédictifs différents qui réalisent un vote majoritaire pour la décision finale. Les forêts aléatoires possèdent naturellement plus d'hyper-paramètres que les arbres de décision, notamment le nombre d'arbre à utiliser ou la possibilité ou non de faire du bootstrap.

La forêt aléatoire utilisée pour la classification des données STRam utilise les hyper-paramètres suivants :

- Bootstrap : Oui
- Critère : Gini
- Nombre d'arbres : 350
- Profondeur maximale : 11
- Nombre maximal de caractéristiques : auto
- Nombre minimal d'échantillon dans un groupe pour séparation : 1
- Random state (ou « clé aléatoire » en français : il s'agit d'un paramètre auquel on fixe une valeur afin que le jeu de données tiré aléatoirement à partir de la base de données soit réutilisé pour chaque forêt aléatoire. Tout résultat aléatoire sera répété à l'identique si la Random state est identique) : 5000 (Le paramètre Random state sert à la répétabilité de l'optimisation du modèle, il n'est pas un paramètre nécessitant d'être optimisé)

Réseau de neurones

Il existe plusieurs typologies de réseaux neuronaux. Ceux que nous avons utilisés pour les modes de réalisation nullement limitatif de l'invention décrits sont des appelés Perceptron Multi-Couche (MLP pour *« Multi-Layer Perceptron »)* et sont basés sur le principe de retro-propagation de l'erreur.

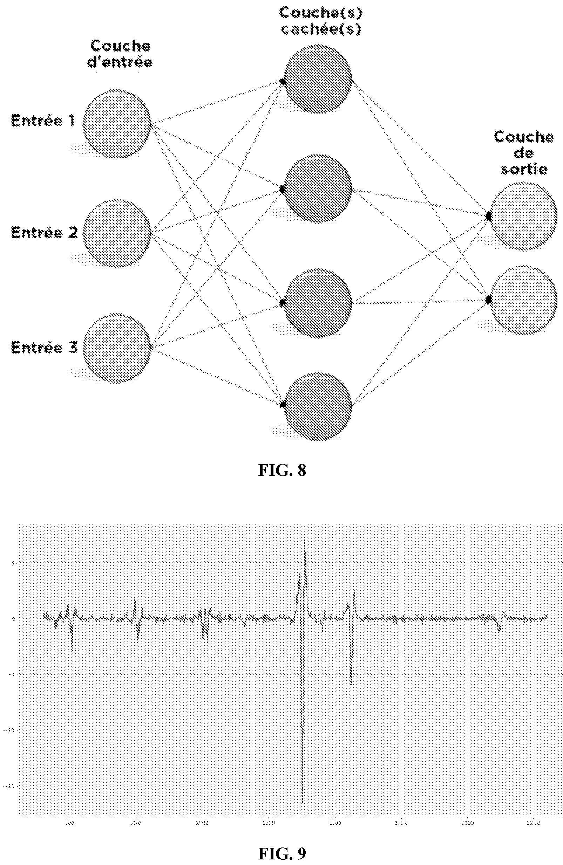

Un MLP est un ensemble d'unités élémentaires, appelés des neurones, établies en différentes couches. La première couche contient autant de neurones qu'il y a de variables explicatives dans le jeu de données (ici, 1959, ou 20 après réduction de dimension), et la dernière contient autant de neurones qu'il y a de labels possibles (ici, 2). Le nombre de couches intermédiaires (également appelées couches cachées) et le nombre de neurones dans ces couches sont des hyper-paramètres du modèle. Tous les neurones d'une couche sont connectés à tous les neurones de la couche suivante. On représente classiquement un réseau de neurones comme illustré dans la figure 8.

Ainsi, un neurone peut être vu comme une fonction mathématique f, appelée fonction d'activation, de la forme :

$$y = f(X_1, \dots, X_N)$$

De plus, la sortie de tous les neurones est pondérée par un coefficient, appelé poids et propre à chaque connexion, qui est un des paramètres optimisés lors de l'apprentissage. Il existe plusieurs méthodes pour optimiser les poids qu'on appelle *« solver »* ou « solutionneur ». Les plus répandues sont les méthodes de descente de gradient mais le choix de cette méthode est également un hyper-paramètre du modèle.

La fonction f est du même type pour tous les neurones des différentes couches cachées et représente un des hyper-paramètres du modèle. Cette fonction est différente pour la couche d'entrée et la couche de sortie et représente également, dans chacun de ces deux cas, un hyper-paramètre du modèle.

Enfin, les paramètres « alpha » (terme de pénalisation) et le taux d'apprentissage (« *learning rate »),* classiquement connus par le spécialiste des réseaux de neurones, sont des hyper-paramètres relatifs à la manière dont l'erreur

commise par le modèle doit impacter l'optimisation des différents poids du réseau lors de la retro-propagation de l'erreur.

Le MLP optimisé pour la classification des données MIRASA résultant de la phase d'optimisation à l'aide de GridSearchCV utilise les hyper-paramètres suivant :

- Fonction d'activation : Sigmoïde
- Alpha : $10^{-5}$
- Nombre de couches cachées : 1
- Nombre de neurones dans la couche cachée : 100
- *« Learning rate »* initial : $10^{-2}$
- *« Learning rate »* : constant
- *« Solver »* : descente stochastique du gradient
- *« Random state »* : 5000

**Prétraitement**

Afin de sélectionner les meilleurs prétraitements spectraux pour les modélisations, il a été développé dans le cadre de la présente invention une grande variété de prétraitements à utiliser sur des spectres des données de spectroscopie Raman exaltée de surface (SERS). Ces prétraitements, qui sont optionnels, comprennent notamment :

- Réduction de moyenne, et/ou
- SNV, et/ou
- Normalisation par le maximum, et/ou
- Normalisation par les extrema, et/ou
- Lissage par algorithme de Savitzky-Golay , et/ou
- Dérivation d'ordre 1 et 2 par algorithme de Savitzky-Golay, et/ou
- Réduction de ligne de base.

Ces prétraitements peuvent être combinés, on peut par exemple effectuer une réduction de ligne de base puis une dérivation des spectres. Cependant, certains prétraitements ne présentent aucun intérêt à être combinés. Par exemple, l'utilisation d'une SNV implique de réaliser une réduction de moyenne. Combiner ces deux prétraitements ne présente donc pas d'intérêt. En ce sens, les moyens logiciels de l'unité 3 prennent en entrée un nombre entier N et génèrent, à partir des prétraitements présents en base, toutes les séries « cohérentes » de N prétraitements possibles, c'est-à-dire les séries ne comportant pas deux prétraitements (ou plus) qui réalisent des opérations similaires, ou encore les séries ne comportant pas deux prétraitements pour lesquels la mise en œuvre de l'un est préjudiciable à la mise en œuvre de l'autre. Une fois cette liste de prétraitements générées, on peut alors entrainer autant de modèles et comparer leurs performances.

Les performances des modèles ont été mesurées par validation, ce qui signifie que les données de la base ont été découpées en un jeu d'entrainement, représentant 80% des données initiales positives et négatives, et un jeu de validation, contenant les 20% restants. Une fois encore, il est important de s'assurer que les spectres issus d'un même patient sont bien tous dans le même groupe soit d'entraînement soit de validation.

La méthode de validation est différente de la méthode du test. Un test consiste à entrainer un modèle et à tester ses performances sur un jeu de données indépendant. Ici, bien qu'on puisse a priori supposer que le jeu de validation est indépendant, on entraine plusieurs modèles et on conserve celui donnant les meilleurs résultats sur ce jeu de validation.

Après étude des différents prétraitements générés par les moyens logiciels de l'unité 3, il a été obtenu que le meilleur prétraitement pour les données MIRASA était un lissage, une correction de ligne de base suivie d'une dérivation d'ordre 2. L'aspect des spectres MIRASA après prétraitement est donné en figure 9.

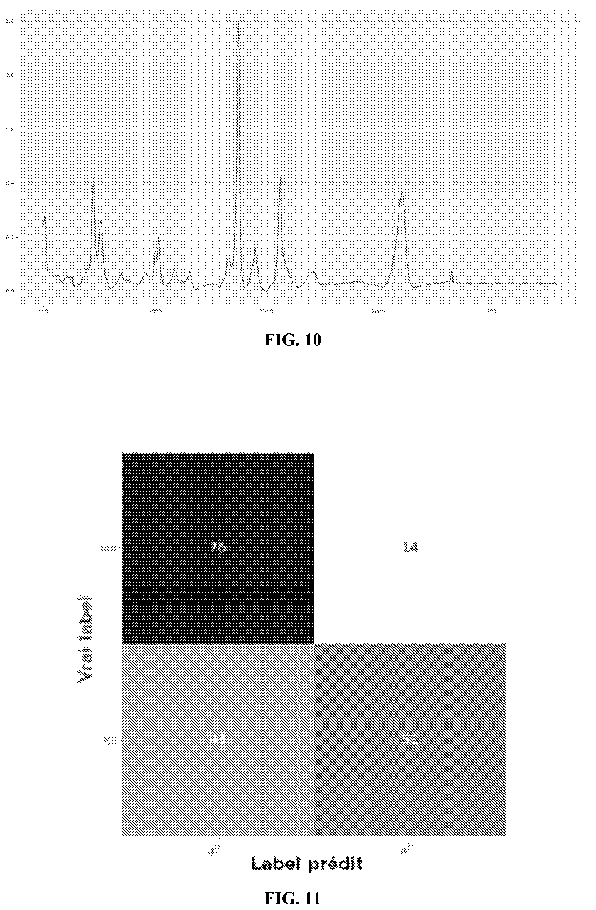

Pour les spectres STRam, comme décrit précédemment, un phénomène de saturation est observé pour certains patients en début de gamme. Ces saturations amenant de la variabilité non désirée dans les données, il a été choisi de supprimer la partie de la gamme spectrale se situant avant $500cm^{-1}$. Après ce choix, le meilleur prétraitement obtenu à l'aide des moyens logiciels de l'unité 3 pour les données STRam correspond à un lissage, une correction de ligne de base et une normalisation par les extrema. La figure 10 présente l'aspect des spectres STRam après prétraitement complet.

**Prédiction**

Les spectres STRam sont tout d'abord prétraités comme mentionné précédemment avant de passer dans chacun des 350 arbres de la forêt aléatoire. Les différents nœuds minimisant le critère de Gini identifiés lors de la phase d'entrainement sont appliqués au spectre et on obtient un label prédit pour chaque arbre. Il y a ensuite vote majoritaire entre 350 prédictions et on obtient le label final prédit par le modèle STRam pour le spectre.

Comme mentionné précédemment, une partie du jeu de données a été mise de côté afin d'optimiser le prétraitement en validation. Il s'agit de 10 patients positifs et 10 patients négatifs correspondant, pour le STRam, à un total de 90 spectres labelisés NEG et 94 spectres labelisés POS. Lorsque l'on applique le modèle à l'intégralité de ces spectres, sans tenir compte de l'appartenance de plusieurs spectres à un même patient, on obtient la matrice de confusion présentée en figure 11.

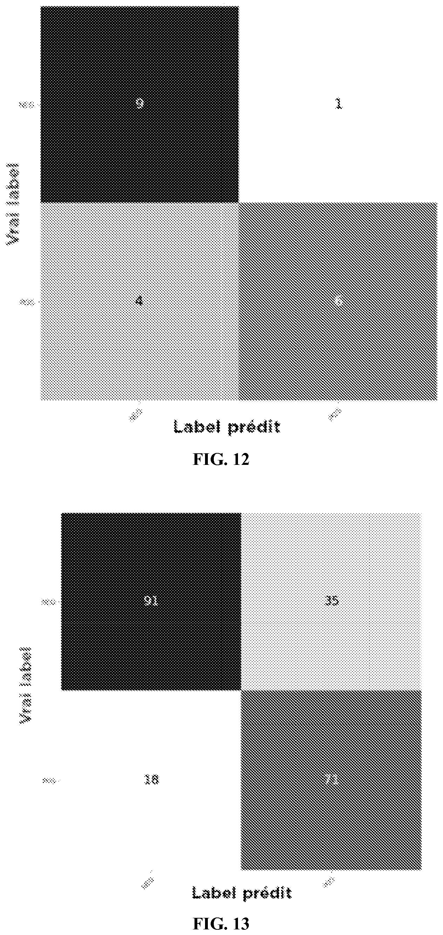

Ces résultats sont associés à une précision globale de 69%, une sensibilité de 54% et une spécificité de 84%. Ces valeurs montrent qu'il est difficile d'identifier correctement les patients POS. Cependant, nous disposons de plusieurs spectres (jusqu'à 9) par patients. Ainsi, en effectuant un vote majoritaire des prédictions faites pour un même patient, on obtient la matrice de confusion de la figure 12.

L'utilisation de plusieurs spectres par patient permet d'obtenir une meilleure prédiction puisqu'on obtient une précision globale de 75%, une sensibilité de 60% et une spécificité de 90%.

De la même manière que pour le STRam, les spectres MIRASA sont d'abord prétraités à l'aide du prétraitement identifié par les moyens logiciels de l'unité 3. Ici, le modèle est un perceptron multi-couche. Ainsi, les données passent successivement dans les différentes couches du réseau neuronal avant d'arriver sur la couche de sortie. Si la couche finale d'un perceptron multi-couche contient, en classification, autant de neurones que de classes à prédire, c'est car chacun de ces neurones est associé à l'un des labels présents dans la base d'entrainement. La classe prédite par un MLP correspond à la classe associée au neurone possédant la plus grande valeur de sortie.

Le jeu de validation pour les données MIRASA est composé de 126 spectres labelisés NEG ainsi que de 89 spectres labelisés POS. Ainsi, la matrice de confusion obtenue en validation est donnée en figure 13.

Cette fois, on obtient une précision globale de 75% pour une sensibilité de 79% et une spécificité de 72%. De la même manière que pour les données STRam, en se servant du fait que chaque patient est associé à plusieurs spectres, on peut obtenir une prédiction globale du modèle MIRASA décrite par la matrice de confusion donnée en figure 14.

De cette manière, on obtient une précision globale de 80%, égale à la sensibilité et la spécificité.

Dans le cadre de l'application de ces modèles à la détection de la présence (ou non) du virus SARS-CoV-2, nous avons pu constater que l'utilisation de plusieurs spectres permettait d'augmenter la robustesse des modélisations. Pour cette raison, il a été décidé que pour la prédiction finale, plusieurs spectres seraient utilisés. Le nombre de 4 spectres a pour le moment été arrêté.

De plus, lors de la prédiction de chaque spectre, il est possible, par construction des modèles, de calculer un pourcentage de certitude du modèle dans sa prédiction. Ainsi, plutôt que d'effectuer un vote majoritaire des 4 spectres, qui pourrait conduire à une égalité, il est préférable selon l'invention de moyenner les probabilités d'appartenance de tous les spectres d'un même patient et de choisir alors le label qui correspond à la probabilité maximum. Cette façon de procéder permet d'assortir à la prédiction finale un indice de confiance égale à la probabilité moyenne que le modèle calcule pour le label prédit. Une réponse assortie d'une fiabilité inférieure à 60% est alors issues de spectres pour lesquels les prédictions étaient incertaines, ce qui permet au logiciel de l'unité 3 de signaler qu'il vaudrait mieux répéter cette mesure plutôt que de se fier à ce résultat. Au-delà de 60%, il y a 2 fois plus de chance que le label prédit soit correct plutôt que faux. Cette barrière de 60% est fixée pour le moment mais pourra être amené à changer.

**Exemple** 1 : Détection de la présence du SARS-CoV-2

1.1. Matériel et méthode

Selon un premier exemple, des prélèvements nasopharyngés sont réalisés chez des personnes.

Les prélèvements sont traités par un tampon de lyse et l'ARN est isolé par adsorption sur une matrice de silice et lavage.

Une solution contenant des particules d'or natives de diamètre moyen de 150 nm à une concentration de 0,15 mg/ml (AUNP-COL de Metrohm) est centrifugée à 18 000 g pendant 1 minute.

30 microlitres de l'échantillon contenant l'ARN purifié sont mis en contact avec le culot de nanoparticules puis l'ensemble est agité pour obtenir un milieu homogène.

Des dépôts de 10 microlitres sont faits sur une lame recouverte de papier d'aluminium et les spectres sont réalisés avec un senseur ST-Ram de chez Metrohm d'une puissance d'environ 500 mW avec une longueur d'onde de 785 nm. Il est utilisé entre 10 et 100 % de sa puissance, avantageusement entre 50 et 100 % de sa puissance. Le temps d'intégration de la mesure est compris entre 15 et 60 secondes.

Selon un deuxième exemple, dont les résultats sont présentés ci-dessous, des prélèvements nasopharyngés sont réalisés chez des personnes et déchargés dans un milieu de transport.

Une solution contenant des particules d'or natives de diamètre moyen de 100 nm à une concentration de 0,15 mg/mL (AUNP-COL de Metrohm) est centrifugée à 800 g pendant 45 minutes.

20 microlitres du milieu de transport contenant l'échantillon de prélèvement nasopharyngé sont mis en contact avec 10 $\mu$L du culot de nanoparticules puis l'ensemble est agité pour obtenir un milieu homogène.

Des dépôts de 10 microlitres sont faits sur une lame d'aluminium et les spectres sont réalisés avec un senseur ST-Ram de chez Metrohm d'une puissance d'environ 500 mW avec une longueur d'onde de 785 nm. Il est utilisé entre 10% et 100 % de sa puissance, avantageusement entre 50% et 100% de sa puissance. Le temps d'intégration de la mesure est compris entre 1 et 30 secondes.

1.2. Résultats :

Les résultats sont présentés à la figure 1.

La méthode selon la présente invention est sensible puisqu'elle permet une bonne classification des patients (voir la colonne sensibilité du tableau de la figure 1) donc elle donne peu de patients faux positifs ; elle est spécifique (voir la colonne spécificité du tableau de la figure 1) donc elle permet de distinguer les patients négatifs. Elle présente un index de Youden très élevé. Cet index est calculé selon la formule suivante (sensibilité + spécificité)-1.

Les pics spécifiques du SARS-CoV-2 sont donnés dans les figures 2 et 3.

La présence du SARS-CoV-2 dans un échantillon pour des nanoparticules d'or est caractérisée par la présence d'un pic entre 560 $cm^{-1}$ et 760 $cm^{-1}$ (typiquement à 660 ou 727 $cm^{-1}$), d'un pic entre 1250 et 1500 $cm^{-1}$ (typiquement à 1374 $cm^{-1}$) et d'un pic entre 2062 $cm^{-1}$ et 2162 $cm^{-1}$ (typiquement à 2100 ou 2112 $cm^{-1}$). On dit alors que le patient chez lequel l'échantillon a été prélevé est positif SARS-CoV-2. En revanche en l'absence de virus dans un échantillon, seul un pic entre 1100 et 1250 $cm^{-1}$ est visible. Le patient est alors déclaré négatif au SARS-CoV-2.

Bien sûr, l'invention n'est pas limitée aux exemples qui viennent d'être décrits et de nombreux aménagements peuvent être apportés à ces exemples sans sortir du cadre de l'invention.

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Méthode de détection d'un agent pathogène dans au moins un signal de spectroscopie Raman exaltée de surface, dit SERS, obtenu à partir d'un échantillon mis en contact avec des nanoparticules métalliques natives non magnétiques, la méthode étant mise en œuvre par un ordinateur et comprenant :

   - une réception de chaque signal de spectroscopie Raman exaltée de surface obtenu à partir de l'échantillon mis en contact avec les nanoparticules métalliques natives non magnétiques ;
   - une mise en œuvre d'un modèle de classification configuré pour associer chaque signal de spectroscopie Raman exaltée de surface reçu à au moins une classe représentative d'une présence ou une absence de l'agent pathogène dans l'échantillon,
   - le modèle de classification étant configuré pour appliquer au moins un traitement relatif à quatre pics : un pic entre 560 $cm^{-1}$ et 760 $cm^{-1}$, un pic entre 1250 et 1500 $cm^{-1}$ et un pic entre 2062 $cm^{-1}$ et 2162 $cm^{-1}$, et un pic entre 1100 et 1250 $cm^{-1}$,
   - l'agent pathogène étant le SARS CoV 2,
   - les nanoparticules métalliques natives non magnétiques étant des particules d'or,
   - la présence du SARS CoV 2 étant **caractérisée par** la présence du pic entre 560 cm-1 et 760 $cm^{-1}$, du pic entre 1250 et 1500 $cm^{-1}$ et du pic entre 2062 $cm^{-1}$ et 2162 $cm^{-1}$,
   - l'absence du SARS CoV 2 étant **caractérisée par** la présence du seul pic entre 1100 et 1250 $cm^{-1}$.

2. Méthode selon la revendication **1,** dans laquelle le modèle de classification comprend au moins un parmi : un réseau de neurones, une forêt aléatoire, une machine à vecteur de support, une machine à vecteur de pertinence, un PLSDA, et/ou un modèle bayésien, de préférence un réseau de neurones et/ou une forêt aléatoire.

3. Méthode selon la revendication **1** ou **2,** comprenant, entre la réception et la mise en œuvre du modèle de classification, une étape de prétraitement de chaque signal de spectroscopie Raman exaltée, l'étape de prétraitement comprenant, de préférence, la mise en œuvre d'au moins un des prétraitements suivants : une réduction de moyenne, une variation normale standard, une normalisation par le maximum, une normalisation par les extrema, un lissage, de préférence un lissage par algorithme de Savitzky-Golay, une réduction ou correction de ligne de base, une dérivation, de préférence une dérivation d'ordre 1 ou 2.

4. Méthode selon l'une quelconque des revendications **1** à **3,** comprenant, entre la réception et la mise en œuvre du

modèle de classification, un choix automatique du modèle de classification utilisé parmi plusieurs modèles de classification prédéterminés en fonction :

- d'une forme de prélèvement de l'échantillon ; et/ou
- d'un modèle de spectromètre délivrant chaque signal de spectroscopie Raman exaltée de surface ; et/ou
- de données relatives au sujet sur lequel a été prélevé l'échantillon ; et/ou
- d'un milieu de transport de l'échantillon ; et/ou
- de l'agent ou des agents pathogène(s) à détecter.

**5.** Méthode selon l'une quelconque des revendications **1** à **4,** dans laquelle la réception de chaque signal de spectroscopie Raman exaltée de surface comprend :

a) la mise en contact dudit échantillon avec des nanoparticules métalliques natives non magnétiques pour obtenir une solution ou une suspension ;
b) le dépôt de ladite solution ou de ladite suspension sur un support ; et
c) la détection de chaque signal de spectroscopie Raman exaltée de surface émis par le dépôt.

**6.** Méthode selon l'une quelconque des revendications **1** à **5,** dans laquelle les nanoparticules métalliques natives non magnétiques présentent un diamètre compris entre 50 et 200 nm, avantageusement compris entre 100 et 200 nm, encore plus avantageusement compris entre 100 et 150 nm.

**7.** Méthode selon l'une quelconque des revendications **1** à **6,** dans laquelle la réception de chaque signal de spectroscopie Raman exaltée de surface comprend :

- une émission de lumière d'excitation, de préférence de longueur d'onde comprise entre 750 et 800 nm, ladite lumière d'excitation atteignant l'échantillon,
- une captation, par un capteur ou spectromètre, de lumière réfléchie, transmise, diffusée ou rétrodiffusée par l'échantillon alors que ladite lumière d'excitation atteint l'échantillon.

**8.** Produit programme d'ordinateur comprenant des instructions de code de programme qui, lorsqu'elles sont exécutées par un ordinateur, mettent en œuvre la méthode selon l'une quelconque des revendications **1** à **4.**

**9.** Kit pour la détection de la présence de l'agent pathogène SARS-Cov-2 dans un échantillon par spectroscopie Raman exaltée de surface, dit SERS, ledit kit comprenant des nanoparticules métalliques natives non magnétiques et un logiciel et/ou des moyens logiciels configuré(s) pour détecter la présence dudit agent pathogène dans ledit échantillon par la mise en œuvre d'une méthode selon l'une quelconque des revendications **1** à **4.**

**10.** Kit selon la revendication **9 ,** dans lequel les nanoparticules métalliques natives non magnétiques présentent un diamètre moyen compris entre 50 et 200 nm, avantageusement compris entre 100 et 200 nm, encore plus avantageusement compris entre 100 et 150 nm.

**11.** Utilisation d'un kit selon l'une quelconque des revendications **9** à **10** pour détecter la présence dudit agent pathogène dans un échantillon par spectroscopie Raman exaltée de surface, dit SERS.

**Patentansprüche**

**1.** Verfahren zum Nachweis eines Krankheitserregers in mindestens einem Signal der oberflächenverstärkten Raman-Spektroskopie, bekannt als SERS, das aus einer Probe gewonnen wird, die mit nichtmagnetischen nativen Metallnanopartikeln in Kontakt gebracht wurde, wobei das Verfahren computergesteuert durchgeführt wird und umfasst:

- Empfangen jedes oberflächenverstärkten Raman-Spektroskopiesignals, das aus der Probe erhalten wurde, die mit den nichtmagnetischen nativen Metallnanopartikeln in Kontakt gebracht wurde,
- Anwenden eines Klassifizierungsmodells, das dazu ausgebildet ist, jedes empfangene oberflächenverstärkte Raman-Spektroskopiesignal mindestens einer Klasse zuzuordnen, die für das Vorhandensein oder Nichtvorhandensein des Krankheitserregers in der Probe repräsentativ ist,
- wobei das Klassifizierungsmodell dazu ausgebildet ist, mindestens eine Verarbeitung in Bezug auf vier Peaks anzuwenden: einen Peak zwischen 560 $cm^{-1}$ und 760 $cm^{-1}$, einen Peak zwischen 1250 und 1500 $cm^{-1}$ und einen

Peak zwischen 2062 $cm^{-1}$ und 2162 $cm^{-1}$ und einen Peak zwischen 1100 und 1250 $cm^{-1}$,
- wobei der Erreger SARS CoV 2 ist,
- wobei die nativen nichtmagnetischen Metallnanopartikel Goldpartikel sind,
- wobei das Vorhandensein von SARS CoV 2 durch das Vorhandensein des Peaks zwischen 560 $cm^{-1}$ und 760 $cm^{-1}$, des Peaks zwischen 1250 und 1500 $cm^{-1}$ und des Peaks zwischen 2062 $cm^{-1}$ und 2162 $cm^{-1}$ gekennzeichnet ist,
- wobei das Fehlen von SARS CoV 2 durch das Vorhandensein des einzigen Peaks zwischen 1100 und 1250 $cm^{-1}$ gekennzeichnet ist.

**2.** Verfahren nach Anspruch 1, wobei das Klassifizierungsmodell mindestens eines der folgenden Elemente umfasst: ein neuronales Netz, einen Zufallswald, eine Support-Vektor-Maschine, eine Relevanzvektor-Maschine, ein PLSDA und/oder ein Bayes'sches Modell, bevorzugt ein neuronales Netz und/oder einen Zufallswald.

**3.** Verfahren nach Anspruch 1 oder 2, das zwischen dem Empfang und der Anwendung des Klassifizierungsmodells einen Schritt zur Vorverarbeitung jedes Signals der oberflächenverstärkten Raman-Spektroskopie umfasst, wobei der Vorverarbeitungsschritt bevorzugt die Anwendung mindestens einer der folgenden Vorverarbeitungen umfasst: Mittelwertreduktion, Standardnormalvariation, Normalisierung durch das Maximum, Normalisierung durch die Extrema, Glättung, bevorzugt Glättung durch den Savitzky-Golay-Algorithmus, Baseline-Reduktion oder -Korrektur, Ableitung, bevorzugt Ableitung 1. oder 2. Ordnung.

**4.** Verfahren nach einem der Ansprüche 1 bis 3, umfassend zwischen dem Empfang und der Anwendung des Klassifizierungsmodells eine automatische Auswahl des verwendeten Klassifizierungsmodells aus mehreren vorbestimmten Klassifizierungsmodellen in Abhängigkeit von:

- einer Form der Probenahme; und/oder
- einem Spektrometermodell, das jedes Signal der oberflächenverstärkten Raman-Spektroskopie liefert, und/oder
- Daten über das Subjekt, von dem die Probe entnommen wurde, und/oder
- einem Transportmedium für die Probe; und/oder
- dem oder den nachzuweisenden Krankheitserreger(n).

**5.** Verfahren nach einem der Ansprüche 1 bis 4, wobei der Empfang jedes oberflächenverstärkten Raman-Spektroskopiesignals umfasst:

a) das Inkontaktbringen der Probe mit nichtmagnetischen nativen Metallnanopartikeln, um eine Lösung oder Suspension zu erhalten,
b) Aufbringen der Lösung oder Suspension auf einen Träger, und
c) Detektieren jedes von der Ablagerung emittierten Signals der oberflächenverstärkten Raman-Spektroskopie.

**6.** Verfahren nach einem der Ansprüche 1 bis 5, wobei die nichtmagnetischen nativen Metallnanopartikel einen Durchmesser zwischen 50 und 200 nm, bevorzugt zwischen 100 und 200 nm, stärker bevorzugt zwischen 100 und 150 nm aufweisen.

**7.** Verfahren nach einem der Ansprüche 1 bis 6, wobei der Empfang jedes oberflächenverstärkten Raman-Spektroskopiesignals umfasst:

- eine Emission von Anregungslicht, bevorzugt mit einer Wellenlänge zwischen 750 und 800 nm, wobei das Anregungslicht auf die Probe trifft,
- eine Erfassung des von der Probe reflektierten, durchgelassenen, gestreuten oder rückgestreuten Lichts durch einen Sensor oder ein Spektrometer, während das Anregungslicht auf die Probe trifft.

**8.** Computerprogrammprodukt, das Programmcodeanweisungen umfasst, die, wenn sie von einem Computer ausgeführt werden, das Verfahren nach einem der Ansprüche 1 bis 4 implementieren.

**9.** Kit zum Nachweis des Vorhandenseins des SARS Cov 2-Erregers in einer Probe mittels oberflächenverstärkter Raman-Spektroskopie, bekannt als SERS, wobei das Kit nichtmagnetische native Metallnanopartikel und eine Software und/oder Softwaremittel umfasst, die dazu ausgebildet ist/sind, das Vorhandensein des Erregers in der Probe durch Implementierung eines Verfahrens nach einem der Ansprüche 1 bis 4 nachzuweisen.

**10.** Kit nach Anspruch 9, wobei die nichtmagnetischen nativen Metallnanopartikel einen durchschnittlichen Durchmesser zwischen 50 und 200 nm, bevorzugt zwischen 100 und 200 nm, stärker bevorzugt zwischen 100 und 150 nm aufweisen.

**11.** Verwendung eines Kits nach einem der Ansprüche 9 bis 10 zum Nachweis des Vorhandenseins des genannten Krankheitserregers in einer Probe mittels oberflächenverstärkter Raman-Spektroskopie, bekannt als SERS.

**Claims**

**1.** A method for detecting a pathogen in at least one surface-enhanced Raman spectroscopy, SERS, signal obtained from a sample brought into contact with non-magnetic native metal nanoparticles, the method being implemented by a computer and comprising:

- a reception of each surface-enhanced Raman spectroscopy signal obtained from the sample brought into contact with the non-magnetic native metal nanoparticles;
- an implementation of a classification model configured to associate each surface-enhanced Raman spectroscopy signal received with at least one class representative of a presence or absence of the pathogen in the sample,
- the classification model being configured to apply at least one treatment relating to four peaks : a peak between 560 $cm^{-1}$ and 760 $cm^{-1}$, a peak between 1250 and 1500 $cm^{-1}$ and a peak between 2062 $cm^{-1}$ and 2162 $cm^{-1}$, and a peak between 1100 and 1250 $cm^{-1}$,
- the pathogen being SARS CoV 2,
- the non-magnetic native metal nanoparticles being gold nanoparticles,
- the presence of SARS CoV 2 being **characterized by** the presence of the peak between 560 cm-1 and 760 $cm^{-1}$, the peak between 1250 and 1500 $cm^{-1}$ and the peak between 2062 $cm^{-1}$ and 2162 $cm^{-1}$,
- the absence of SARS CoV 2 being **characterized by** the presence of the only peak between 1100 and 1250 $cm^{-1}$.

**2.** The method according to claim **1,** wherein the classification model comprises at least one among: a neural network, a random forest, a support vector machine, a relevance vector machine, a PLSDA, and/or a Bayesian model, preferably a neural network and/or a random forest.

**3.** The method according to claim **1** or **2,** comprising, between the reception and the implementation of the classification model, a preprocessing step of each surface-enhanced Raman spectroscopy signal, the preprocessing step comprising, preferably, the implementation of at least one of the following pretreatments: a reduction of average, a standard normal variation, normalization by the maximum, normalization by extrema, smoothing, preferably smoothing by a Savitzky-Golay algorithm, reduction or correction of baseline, a derivation, preferably a derivation of order 1 or 2.

**4.** The method according to any one of claims **1** to **3,** comprising, between the reception and the implementation of the classification model, an automatic choice of the used classification model from several predetermined classification models based on:

- a form of sample collection; and/or
- a spectrometer model delivering each surface-enhanced Raman spectroscopy signal; and/or
- data relating to a subject from whom the sample was taken; and/or
- a sample transport medium; and/or
- the pathogen or the pathogen(s) to be detected.

**5.** The method according to any one of claims **1** to **4,** wherein the reception of each surface-enhanced Raman spectroscopy signal comprises:

a) the contact between said sample and non-magnetic native metal nanoparticles to obtain a solution or a suspension;
b) the deposit of said solution or said suspension on a support; and
c) the detection of each surface-enhanced Raman spectroscopy signal emitted by the deposit.

6. The method according to any one of claims **1** to **5,** wherein the non-magnetic native metal nanoparticles have a diameter between 50 and 200 nm, advantageously comprised between 100 and 200 nm, more advantageously comprised between 100 and 150 nm.

7. The method according to any of claims **1** to **6,** wherein the reception of each surface-enhanced Raman spectroscopy signal comprises:

   - an emission of excitation light, preferably of wavelength between 750 and 800 nm, said excitation light reaching the sample,
   - a capture, by a sensor or a spectrometer, of a reflected, transmitted, scattered or backscattered light by the sample while said excitation light reaches the sample.

8. A computer program product comprising program code instructions which, when executed by a computer, implement the method according to any one of claims **1** to **4.**

9. A kit for detecting the presence of the pathogen SARS-Cov-2 in a sample by surface-enhanced Raman spectroscopy, SERS, said kit comprising non-magnetic native metal nanoparticles and a software and/or software means configured to detect the presence of said pathogen in said sample by implementing a method according to any one of claims **1** to **4.**

10. The kit according to claim **9,** wherein the non-magnetic native metal nanoparticles have an average diameter between 50 and 200 nm, advantageously comprised between 100 and 200 nm, more advantageously comprised between 100 and 150 nm..

11. Use of a kit according to any one of claims **9** to **10** for detecting the presence of said pathogen in a sample by surface-enhanced Raman spectroscopy, SERS.

| Echantillon | Score d'entraînement | Score de test | Sensibilité | Spécificité |
|---|---|---|---|---|
| ARN | 91,1 % | 87,5 % | 91,7 % | 83,3% |
| ARN | 100 % | 75 % | 75 % | 75 % |
| ARN | | | 91,7 % | 100 % |
| ARN | 100 % | 80 % | 66,7 % | 87,3 % |
| Inactivé | | 87,5 % | 100 % | 75 % |
| Inactivé | | 92 % | 83 % | 100 % |
| Inactivé | | | 92 % | 100 % |
| Inactivé | | | 100 % | 92 % |

FIG. 1

covid19
NEG
POS

FIG. 2

POS -> +, NEG -> x, Au -> blue, Ag -> green
7000
6000
5000
4000
3000
2000
1000
500
750
1000
1250
1500
1750
2000
2250

FIG. 3

2
10
Spectromètre
Réseau de diffraction
Détecteur
1
Laser
Filtre *notch*
3
Microscope optique
Échantillon
4

FIG. 4

Intensité
Décalage Raman (cm-1)

FIG. 5

A
Décalage Raman (cm-1)

B
Décalage Raman (cm-1)

FIG. 6

True
False

FIG. 7

Couche
d'entrée
Couche(s)
cachée(s)
Couche
de
sortie
Entrée 1
Entrée 2
Entrée 3

FIG. 8

FIG. 9

FIG. 10

76
14
Vrai label
Label prédit

FIG. 11

9
1
4
6
Vrai label
Label prédit

FIG. 12

91
35
18
71
Vrai label
Label prédit

FIG. 13

8
2
2
8
Vrai label
Label prédit

FIG. 14

Répartition des pics
101
102
103
104
105
106
107
109
110
111
112
Décalage Raman (cm⁻¹)

FIG. 15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2021203041 A1
- EP 3901617 A1


### Littérature non-brevet citée dans la description


- optofluidic label-free SERS platform for rapid bacteria detection in serum. **HUNTER ROBERT et al.** sensors and actuators b: chemical. Elsevier bv, 31 July 2019
- SERS based point of care detection of food-borne pathogens. **MUNGROO NAWFAL ADAM et al.** mikrochimica acta. springer verlag, 15 December 2015